# EUROPEAN PATENT APPLICATION

(11) **EP 2 574 627 A1**
(43) Date of publication of application: **03.04.2013**
(21) Application number: 11007951.4
(22) Date of filing: 30.09.2011
(51) Int. Cl.: C07K 16/28, G01N 33/574

(54) **Particular uses of CD24 inhibitors**

(71) Applicant: Deutsches Krebsforschungszentrum, Stiftung des öffentlichen Rechts, 69120 Heidelberg (DE)
(72) Inventor: Bretz, Niko, 69121 Heidelberg (DE); Altevogt, Peter, 69151 Neckargemünd (DE); Moldenhauer, Gerhard, 69120 Heidelberg (DE); Salnikov, Alexei V., 69126 Heidelberg (DE); Schlange, Thomas, 42781 Haan (DE)
(74) Representative: Lahrtz, Fritz

(57) **Abstract**

The present invention relates to a CD24 inhibitor for use in a method for treating a neoplastic condition in a subject, wherein said subject has been identified by means of a method of the invention. Similarly, the invention relates to a CD24 inhibitor for use in a method for treating a neoplastic condition in a subject, comprising i) treating a sample of said subject with a CD24 inhibitor; ii) determining one or more predictive levels in the sample and iii) administering a CD24 inhibitor to said subject in dependence of said predictive levels.

## Description

### FIELD OF THE INVENTION

The present invention, among others, relates to the fields of CD24 and CD24 inibitors and to the use of same or components related to same, respectively, in the treatment of tumors and other medical applications.

### BACKGROUND OF THE INVENTION

CD24 is a (mucin-like) highly N- and O- glycosylated, glycosylphosphatidylinositol (GPI)-anchored membrane protein of 31 amino acids in humans and 27 in mice (Kay et al., 1991), (Kay et al., 1990), (Wenger et al., 1991). Due to its extensive decoration with carbohydrates, human CD24 appears by SDS-PAGE with a molecular weight of 30-70 kDa (Kristiansen et al., 2010a). Human CD24 has a protein core of only 31 amino acids with 16 potential O- and N-glycosylation sites with mucin-like characteristics (Kristiansen et al., 2004).

CD24 was discovered as a lymphoid differentiation marker in the mouse being expressed on hematopoietic cells such as activated T cells, B cells, macrophages, dendritic cells and neutrophils, but also being present in the developing brain as well as on certain epithelial cells (Fang et al., 2010). CD24 in mouse and humans was identified as a ligand for P-selectin, an adhesion receptor on endothelial cells and platelets (Sammar et al., 1994), (Aigner et al., 1997), (Friederichs et al., 2000). In addition, in tumor cells, CD24 has been identified as a ligand of P-selectin that supports the rolling of breast carcinoma cells on endothelial cells and adhesion to platelets, and might therefore facilitate the metastatic spread of tumor cells (Aigner et al., 1998; Aigner et al., 1997; Friederichs et al., 2000; Baumann et al., 2005; Kristiansen et al., 2004).

In normal tissues, human CD24 is expressed by B-cell precursors, neutrophils, neuronal cells and certain epithelial cells (Kristiansen et al., 2004).

CD24 knockout mice were found to be viable but displayed a mild block in bone marrow maturation of B lymphocytes (Nielsen et al., 1997). Pre-B cell lines established from CD24 knockout mice showed reduced α4-integrin mediated cell binding to activated endothelioma cells as well as fibronectin and this defect was restored by re-expression of CD24 (Hahne et al., 1994). An impact on integrin-mediated cell binding was also noted in carcinoma cell lines that overexpressed CD24 (Baumann et al., 2005).

Due to its GPI-anchor, CD24 was described to be exclusively localized in detergent-resistant membrane domains (DRMs) that have also been termed "lipid-rafts" (Schabath et al., 2006) (Runz et al., 2008). These membrane microdomains are considered important signaling platforms, i.e. platforms for signaling molecules such as G-proteins and Src family protein-tyrosine kinases (Varma et al., 1998) (Simons et al., 2010). Signaling molecules such as G-proteins, Src family protein-tyrosine kinases, and GPI-anchored membrane proteins like CD24 reside in opposite leaflets of the same sphingolipid-enriched microdomains (Ilangumaran et al., 1999).

Indeed, a physical interaction of CD24 with members of the family of Src-kinases, likely mediated by lipid rafts, has been observed (Zam et al., 1996) (Sammar et al., 1997) (Stevanova et al., 1991). Two recent reports have suggested that CD24 signals via Src-kinases in human tumors. Baumann et al. found that CD24 interacts and promotes the activity of c-Src within lipid rafts in breast cancer cells and thereby increases integrin-dependent adhesion (Baumann et al., 2011). In addition, the overexpression of CD24 augmented contractile forces necessary for cell invasion that was blocked by knock-down of the β1-integrin or in the presence of inhibitors to myosin light chain or Src (Mierke et al., 2011). These molecular (CD24-Src) associations could possibly be of functional relevance for other CD24-mediated downstream signaling / effects, but they have not yet been fully investigated.

Interestingly, CD24 is often highly expressed in various human (solid) tumors (e.g. lung, brain, stomach, colorectal, prostate, breast and ovarian) and its expression has been associated with poor prognosis (Kristiansen et al., 2004) (Kristiansen et al., 2010a) (Lim, 2005). Recently, CD24 has been introduced as a marker for normal and cancer stem cells, although this is controversial and under intensive investigation (Woodward and Sulman, 2008).

Experimentally, the overexpression or depletion of CD24 alters cellular functions like proliferation, adhesion and invasion *in vitro* and tumor growth *in vivo.* Generally, experimental work during the last decade has provided evidence for a role of CD24 in tumor (cell) growth, adhesion, migration, invasion, proliferation and metastatic tumor spread, and provides evidence that it alters gene expression in colon and pancreatic cell lines (Ahmed et al., 2009) (Aigner et al., 1998) (Baumann et al., 2005) (Baumann et al., 2011) (Fukushima et al., 2007) (Hahne et al., 1994) (Mierke et al., 2011) (Overdevest et al., 2011) (Runz et al., 2008) (Sagiv et al., 2008) (Schabath et al., 2006) (Senner et al., 1999) (Smith et al., 2006) (Wang et al., 2010). Recently, CD24 was also proposed as a marker for cancer stem cells, although this is controversial and under intensive investigation (Al-Hajj et al., 2003), (Woodward et al., 2008). Other recent studies have suggested that CD24 is a target gene for the transcription factor tGLI1 (truncated glioma-associated oncogene homolog 1) in glioblastoma and breast cancer cells (Lo et al., 2009), (Cao et al., 2011). Another recent report has also suggested a role of CD24 in mRNA stability in cancer cells (Taniuchi et al., 2011).

Tissue factor pathway inhibitor-2 (TFPI-2) is a member of the Kunitz-type serin proteinase inhibitor family and is secreted into the ECM (Konduri et al., 2000), (Herman et al., 2001). It inhibits the tissue factor/factor VIIa (TF/VIIa) complex and a wide variety of serine proteinases including plasmin, plasma kallikrein, factor XIa, trypsin, and chymotrypsin (Konduri et al., 2001), (Konduri et al., 2001), (Gaud et al., 2009). In addition, TFPI-2 has been shown to impair the activity of several matrix metalloproteases (MMP-1, MMP-2, MMP-3, MMP-9 and MMP-13) (Herman et al., 2001). The production of TFPI-2 is reduced or absent during tumor progression, whereby TFPI-2 is involved in the maintenance of the integrity of the tumor microenvironment and inhibits invasiveness, metastases formation and angiogenesis of neoplastic tissue (Sierko et al., 2007). Expression of TFPI-2 has been shown to correlate inversely with an increasing degree of malignancy (Sierko et al., 2007). Therefore, TFPI-2 was described as a tumor suppressor gene that, with its potent inhibitory functions, is able to influence the invasive and metastatic potential of tumor cells (Sierko et al., 2007).

Due to its pleiotropic expression, the cellular function of CD24 has raised interests of researchers in diverse fields such as immunology, tumor biology and stem cell biology. Yet, despite the studies referred to above, it is largely unknown how and by which (cellular) mechanisms CD24 can mediate such effects and regulate such diverse cellular functions, and the functional role of the CD24 molecule is still ill defined. In any case, is remarkable that CD24 as a non-transmembrane molecule is endowed with signaling capacity.

Hence, there is always a need in the art for improved tumor therapies as well as methods for predicting the susceptibility of tumor patients for and their responsiveness to given therapies.

### SHORT DESCRIPTION OF THE FIGURES

In the present figures and description, among others the following abbreviations are used: BrCa: breast carcinoma; ECM: extracellular matrix; GAPDH: glyceraldehyde 3-phosphate dehydrogenase; GPI: glycosylphosphatidylinositol; mAb: monoclonal antibody; pAb: polyclonal antibody; qPCR: quantitative real-time PCR; siCD24: siRNA specific for CD24; siGFP: siRNA specific for GFP (green fluorescent protein); TFPI-2: Tissue Factor Pathway Inhibitor-2; TMA: tissue microarray.

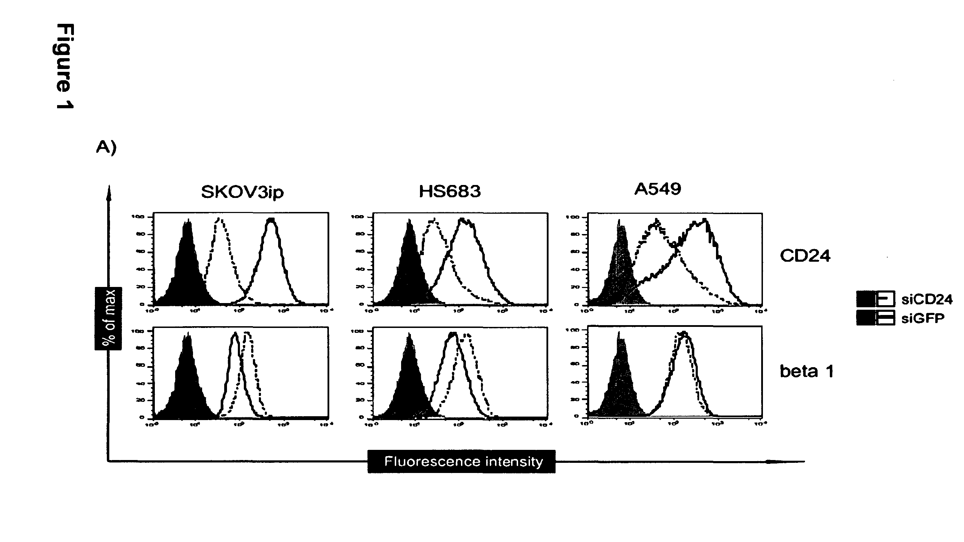

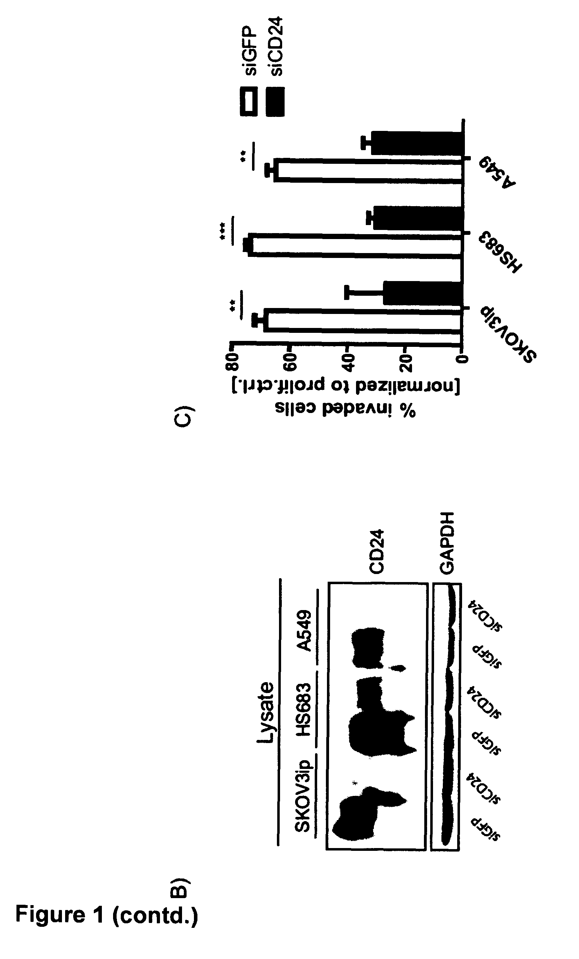

**Fig. 1** shows that CD24 knock-down reduces invasion in human tumor cells. (**A**) The indicated tumor cell lines were subjected to siRNA-mediated knock-down of CD24. Cells were stained with antibodies to CD24 (SWA11) or p1-integrin followed by PE-conjugated goat anti mouse IgG. (**B**) Western blot analysis of tumor cell lysates using SWA11. (C) Matrigel invasion assay was used to determine the invasive potential of CD24 knock-down cells. Shown is the percentage of invaded cells normalized to proliferation control. For (B) and (C) representative results of n=4 experiments are shown.

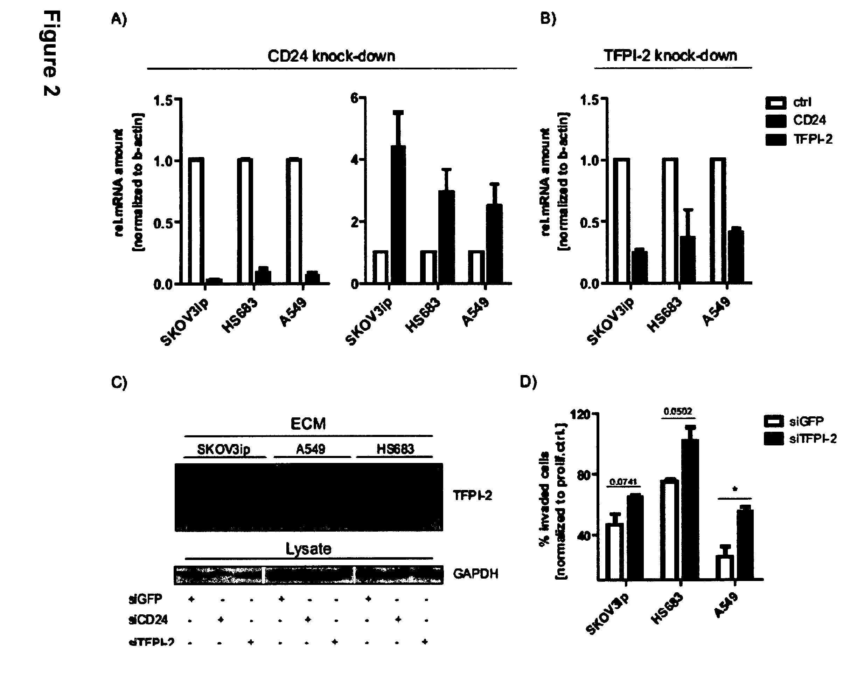

**Fig. 2** illustrates that TFPI-2 is regulated in a CD24 dependent manner and is able to influence tumor cell invasion. (**A and B**) qRT-PCR analysis of CD24 and TFPI-2 expression after CD24 or TFPI-2 specific siRNA-mediated knock-down. siGFP was used as internal control to calculate relative expression values. (**C**) Deposition of TFPI-2 protein in the ECM. Equal numbers of siRNA treated cells were seeded and cultured for 48h. After lysis of the cells the ECM was eluted from the dish and analyzed by Western blot analysis using pAb to TFPI-2. TFPI-2 shows multiple bands between 25-35 kDa. GAPDH in the cell lysate was used to demonstrate equal plating of cells. (**D**) Matrigel invasion assay was performed with TFPI-2 knock-down cells. The data presented are pooled results of n=4 experiments (A, B) and representative results of n=3 experiments are shown in (C) and (D)

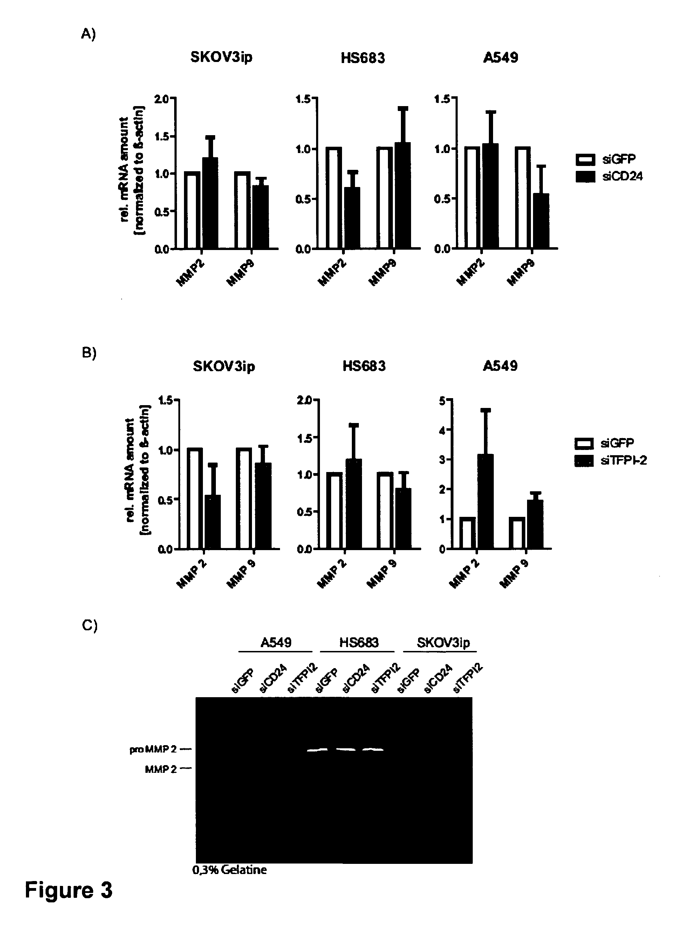

**Fig. 3** illustrates the effect of CD24 or TFPI-2 siRNAs on MMP-2 and MMP-9 expression. (**A and B**) qRT-PCR analysis of MMP-2 and MMP-9 expression after CD24 or TFPI-2 specific siRNA-mediated knock-down. siGFP was used as internal control to calculate relative expression values. (**C**) Proteolytic activity of tissue culture supernatant from the indicated cell lines after siRNA knock-down was tested using gelatine zymography. After incubation for 18 8 hours in developing buffer, the gel was stained with Coomassie brilliant blue R-250. Mature and pro-MMP-9 are undetectable and the positions of MMP-2 and pre-MMP-2 are indicated.

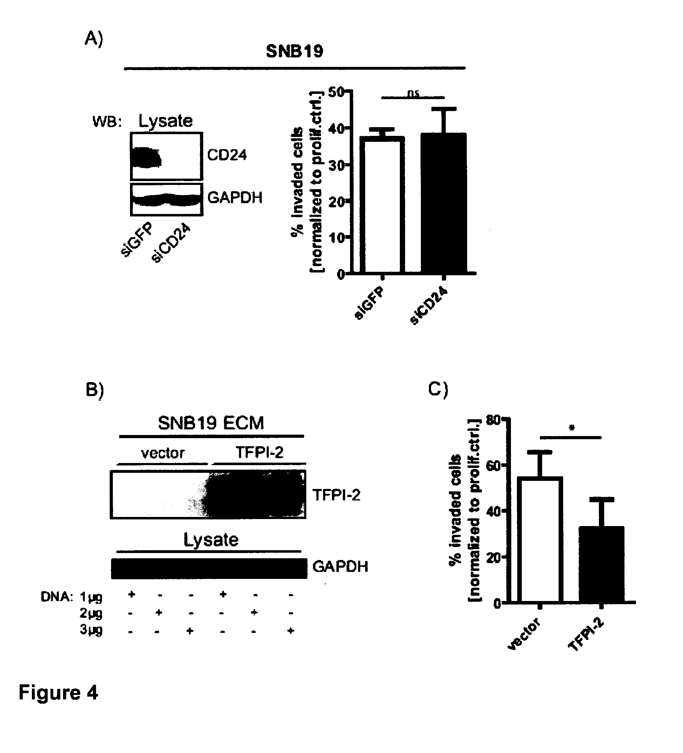

**Fig. 4** shows that transient overexpression of TFPI-2 results in reduced SNB19 cell invasion. (**A**) The TFPI-2 negative cell line SNB19 was depleted for CD24 using specific siRNA and examined for invasive capacity in a Matrigel invasion assay. (**B**) Transient overexpression of a TFPI-2 expression plasmid in SNB 19 cells was monitored by the analysis of the ECM by Western blot analysis using pAb to TFPI-2. GAPDH in the cell lysate was used to demonstrate equal plating of cells. (**C**) Matrigel invasion assay after TFPI-2 overexpression in SNB 19 cells. Representative results of n=3 experiments are shown.

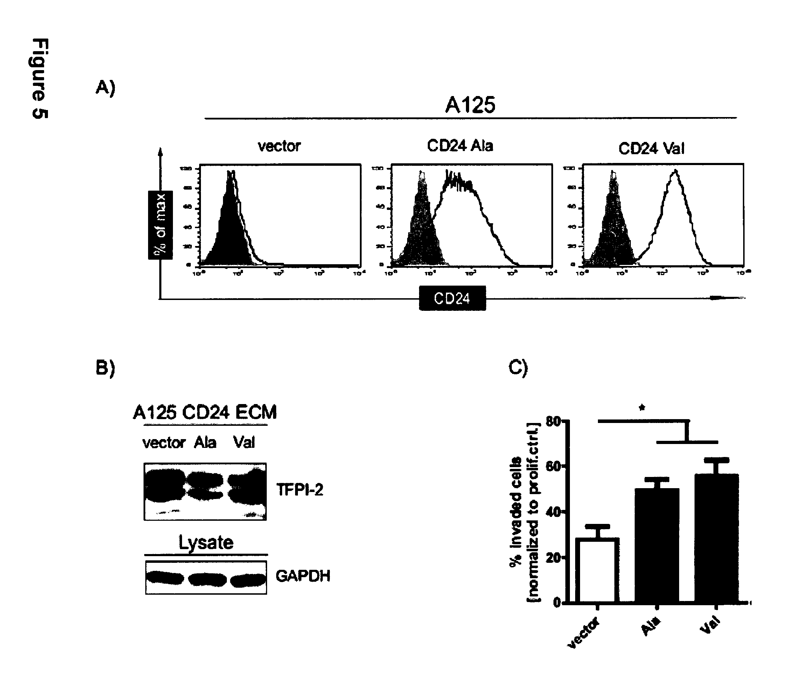

**Fig. 5** illustrates that stable overexpression of CD24 in A125 cells reduced TFPI-2 in the ECM and enhanced cellular invasive potential. (**A**) Cytofluorographic analysis of A125 stably transfected with empty vector, CD24-Ala or CD24-Val forms. Cells were stained with antibodies to CD24 (SWA11) followed by PE-conjugated goat anti mouse IgG. (**B**) Cells were seeded at equal density and the ECM was analyzed by Western blotting with a pAb to TFPI-2 after 48 h. GAPDH in the cell lysate was used to demonstrate equal plating of cells. (**C**) Matrigel invasion assay of A125 cells. A representative experiment of n=3 is shown.

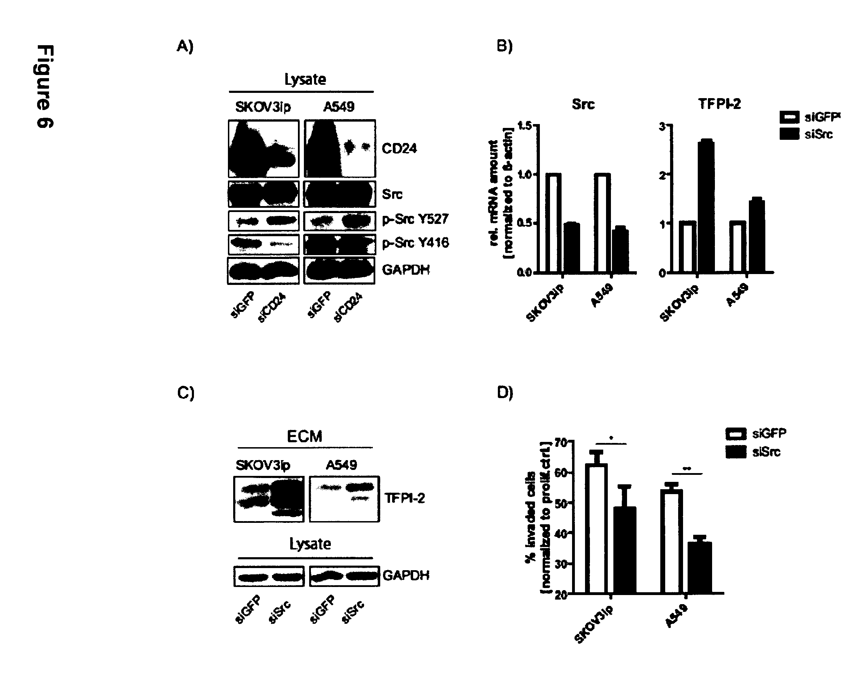

**Fig. 6** depicts Src regulates TFPI-2 protein distribution and affects tumor cell invasion. (**A**) The indicated tumor cell lines were subjected to siRNA-mediated knock-down of CD24. Total cell lysates were analyzed for phosphorylation status of Src kinase by Western blotting. (**B**) Src specific siRNA treated cells were analyzed for TFPI-2 and Src by qRT-PCR. (**C**) Deposition of TFPI-2 protein in the ECM. Equal numbers of siRNA treated cells were seeded and cultured for 48 h. After lysis of the cells the ECM was eluted from the dish and analyzed by Western blot analysis using pAb to TFPI-2. GAPDH in the cell lysate was used to demonstrate equal plating of cells. (**D**) Matrigel invasion assay of Src siRNA treated cells. Representative results of n=3 experiments are shown.

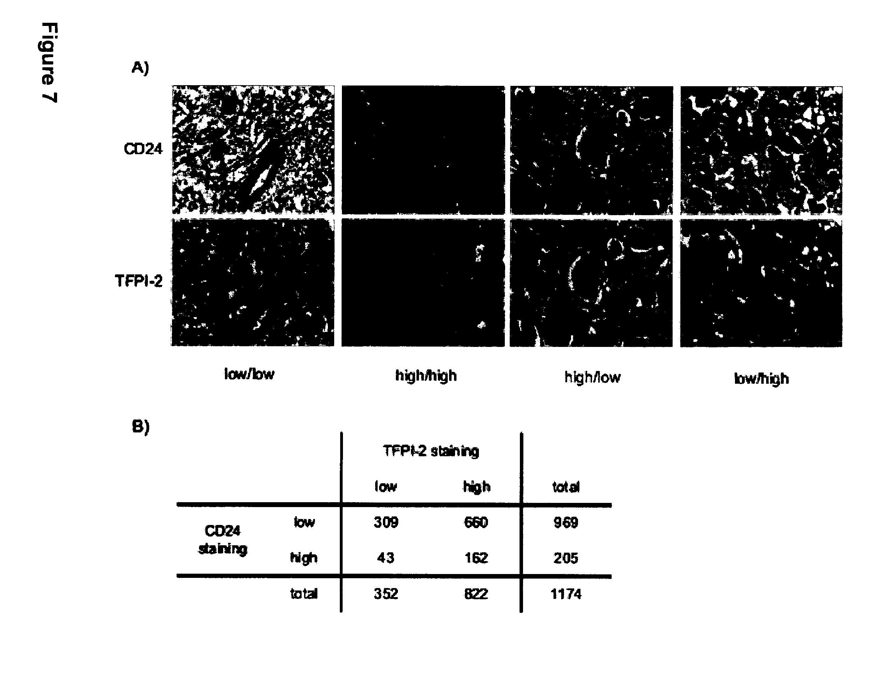

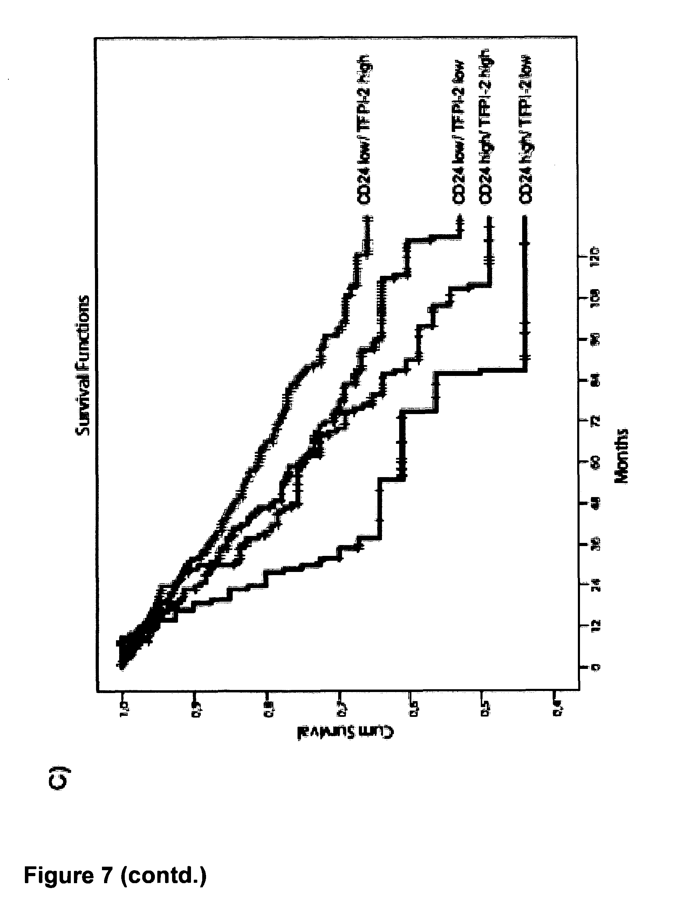

**Fig. 7** illustrates the expression and clinical significance of CD24 and TFPI-2 expression in primary BrCa. (**A**) Staining examples and Scoring of BrCa tumor sections after CD24 and TFPI-2 staining of the TMA. (**B**) Crosstable analysis of patients in the study showing the relation of CD24 and TFPI-2 staining. (**C**) Survival analysis of BrCa patients with the indicated tumor phenotypes.

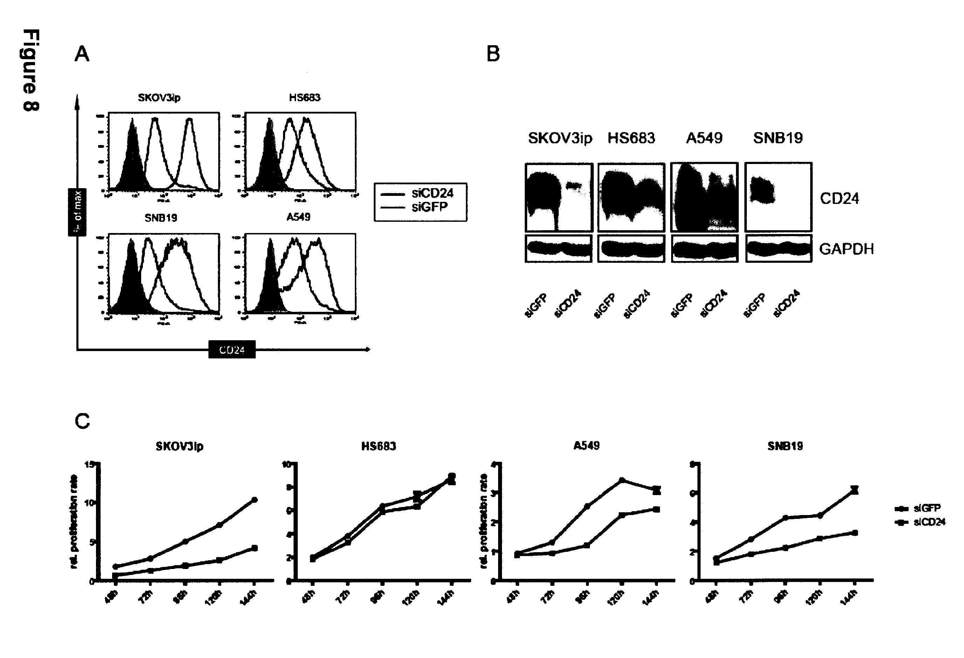

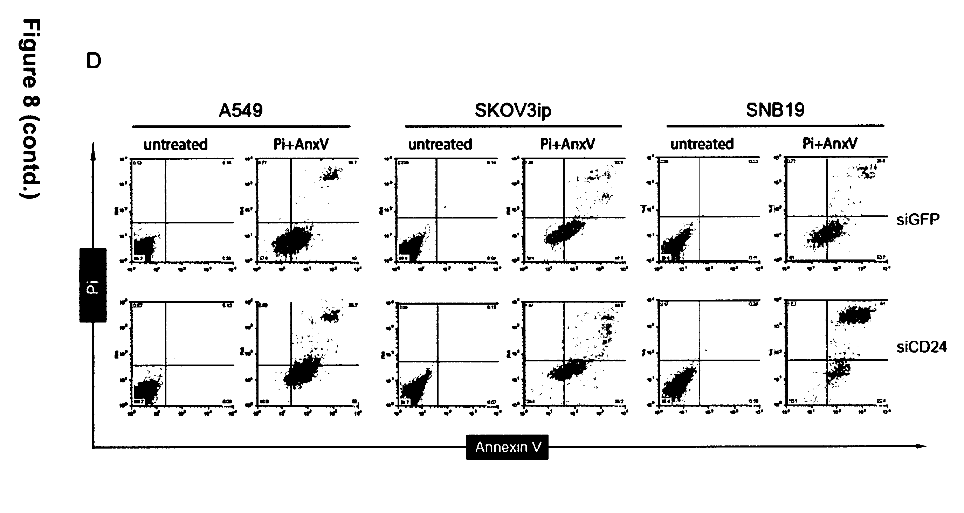

**Fig. 8** shows that knock-down of CD24 decreases cell proliferation. (**A**) The indicated tumor cell lines were subjected to siCD24-mediated knock-down of CD24. Cells were stained with antibodies to CD24 (SWA11) followed by PE-conjugated goat anti mouse IgG. (**B**) Western blot analysis of tumor cell lysates using SWA11. (C) MTT proliferation assay of cells after siCD24 knock-down. Proliferation was tested at the indicated time points. (**D**). Induction of apoptosis after siCD24-1 knock-down using PI- and Annexin-V staining. The analysis was carried out at the 96 h time point.

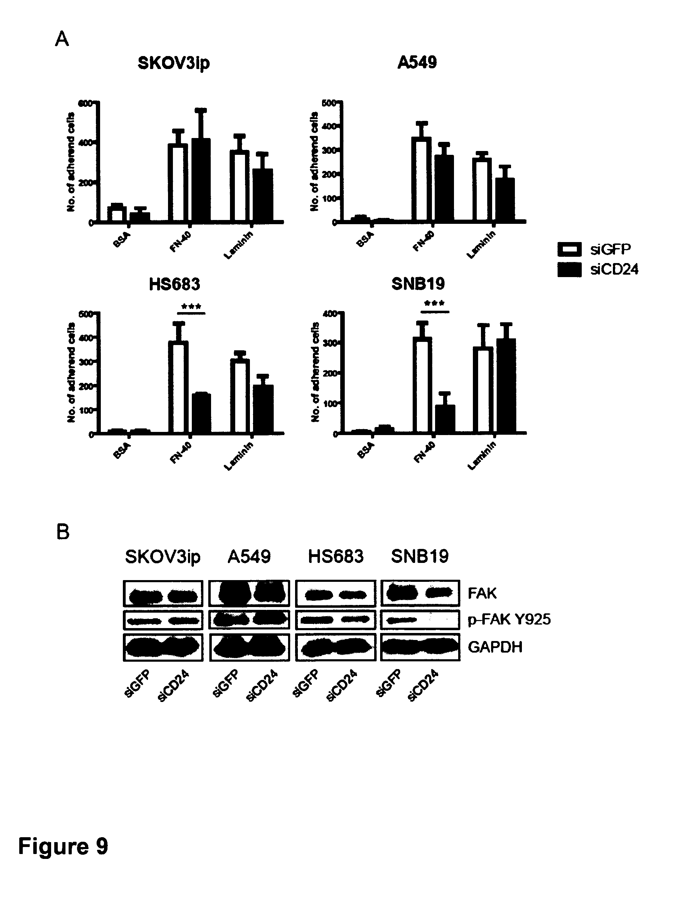

**Fig. 9** illustrates that CD24 knock-down alters cell adhesion and FAK phosphorylation (**A**) The indicated tumor cell lines were subjected to siCD24-mediated knock-down of CD24. Cells were analyzed for cell binding to immobilized substrates FN-40, laminin and BSA for background control. (**B**). Analysis of FAK phosphorylation in siCD24-depleted cells. Cell lysates of the indicated cells were tested for FAK and the FAK-Y925 (Src-dependent) phosphorylation sites.

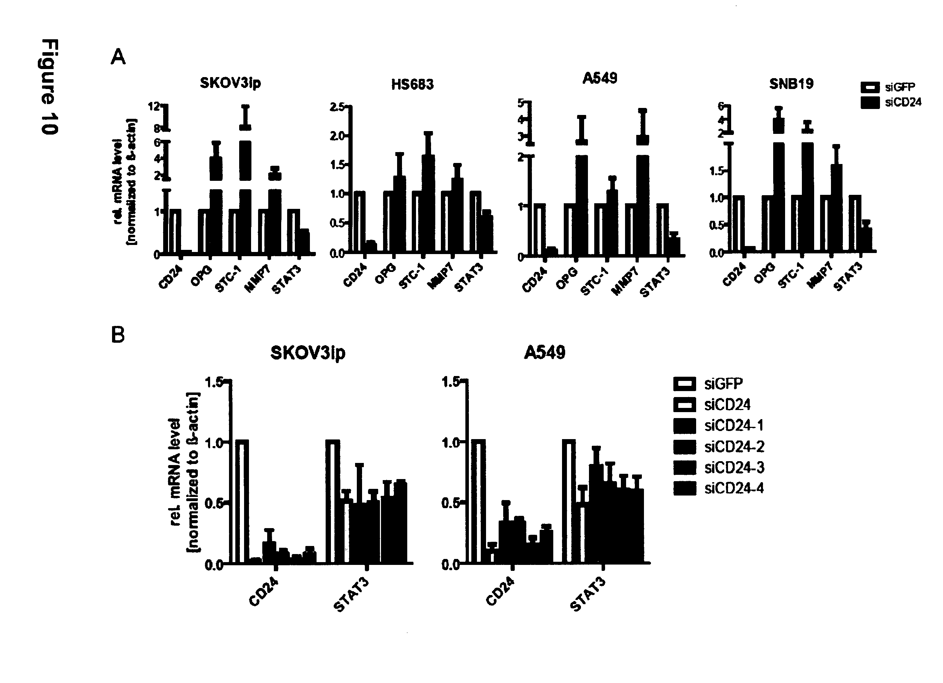

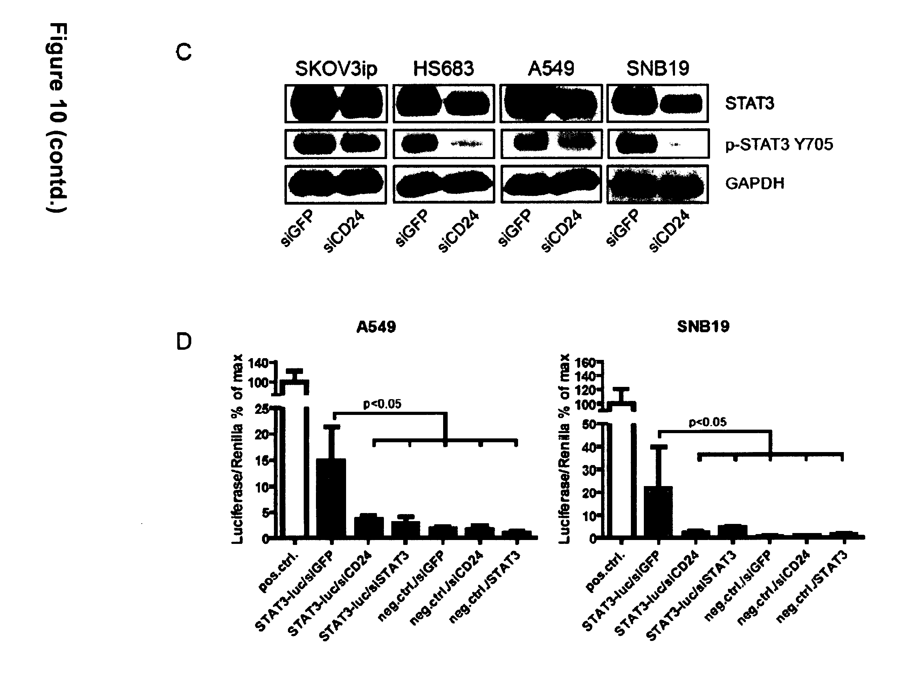

**Fig. 10** illustrates that CD24 knock-down mediates gene regulation and reduces STAT3 protein phosphorylation and activity. (**A**) Identification of a CD24-dependent target genes signature (OPG, STC-1, MMP-7 and STAT3). RT-PCR analysis of mRNAs isolated after si-CD24-1 mediated knock-down. OPG, STC-1 and MMP-7 are up-regulated whereas STAT3 is down-regulated in the indicated cell lines. (**B**) Analysis of STAT-3 levels after siRNA-mediated knock-down of CD24. All oligonucleotides specific for CD24 reduce STAT3 expression levels. (**C**) Western blot analysis for STAT3 and phospho-STAT3 after siCD24-mediated depletion of CD24 (left panel). Right panel: Overexpression of CD24 in A125 cells augments phospho-STAT3 (Y705) levels. CD24-Ala and CD24-Val are isoforms of CD24 differing in the amino acid in the GPI-anchor. (**D**) Decreased STAT3 activity after CD24 or STAT3 knock-down (as a positive control) was measured using a STAT3 specific luciferase-reporter assay.

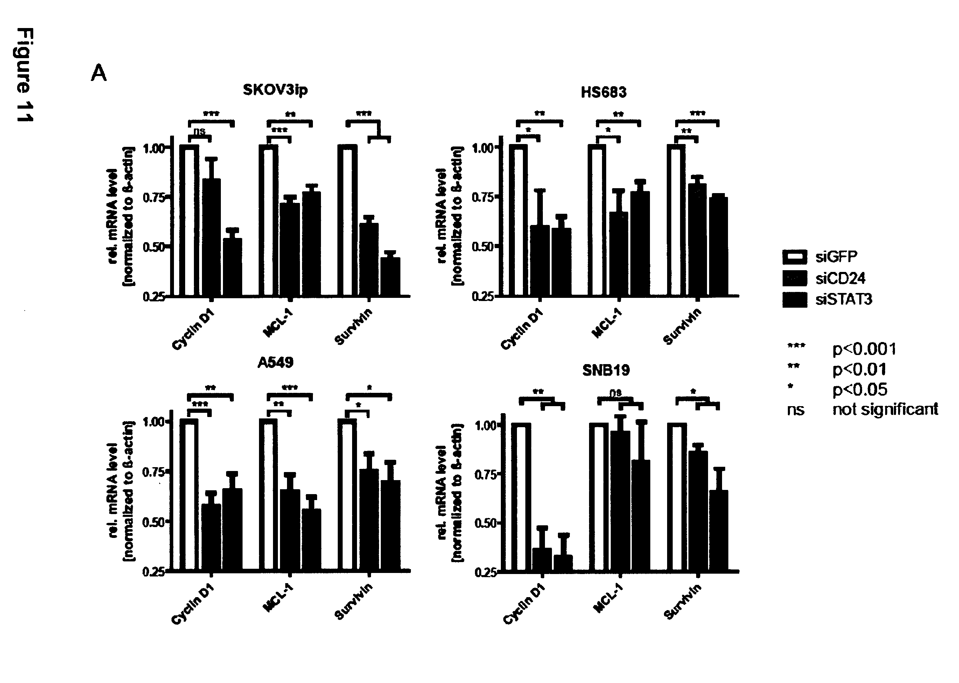

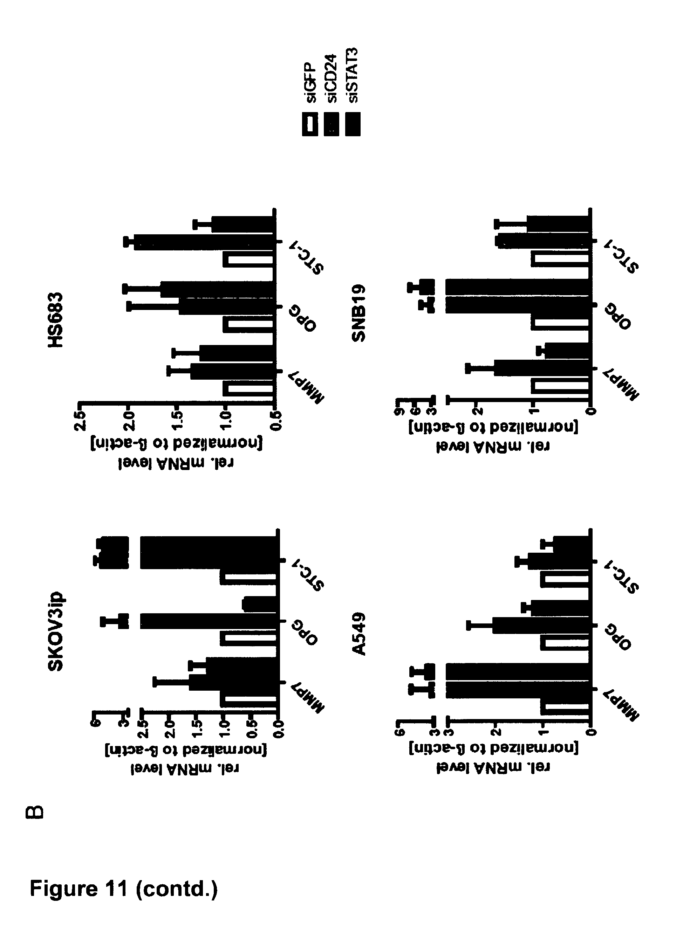

**Fig. 11** illustrates that CD24-knock-down affects activity of classical STAT3 target genes (**A**) Known STAT3 target genes (cyclin D1, MCL-1, survivin) were analyzed by RT-qPCR after CD24 or STAT3 knock-down in the indicated tumor cell lines. Relative mRNA levels normalized to ß-actin were compared to siGFP control. Pooled results of n=4 experiments are shown. (**B**) Regulation of the CD24-dependent target gene signature expression after STAT3 silencing was determined by qRT-PCR. Relative mRNA levels normalized to ß-actin were compared to siGFP control. Pooled results of n=4 experiments are shown.

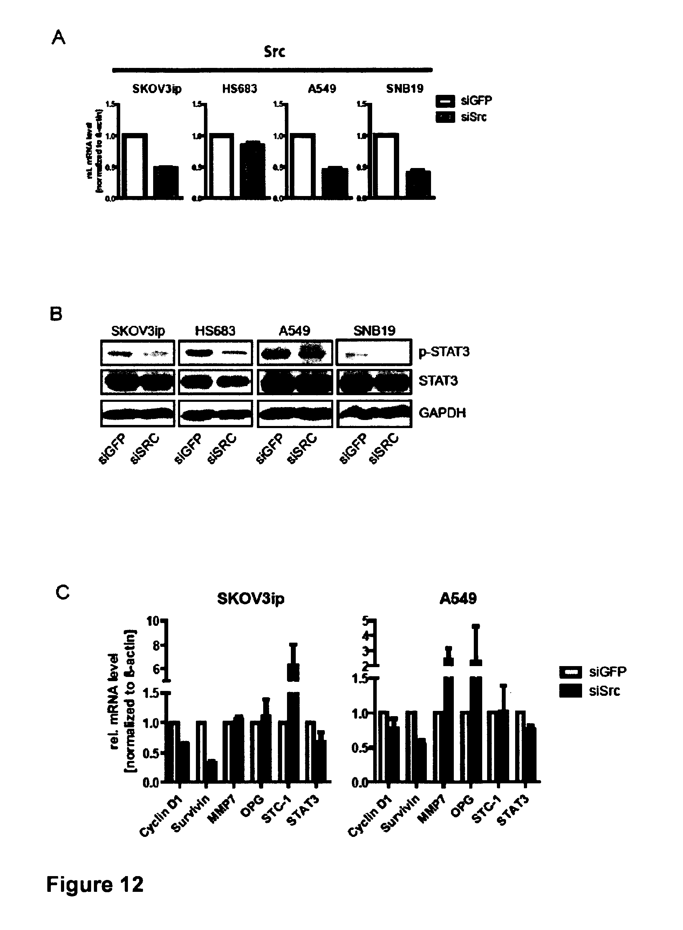

**Fig. 12** shows that Src-silencing affects phosphorylation of STAT3 and STAT3 target gene regulation. (**A**) The indicated tumor cell lines were subjected to siRNA-mediated knock-down of Src and probed by pRT-PCR for knock-down efficacy. (**B**) Phosphorylation of STAT3 after Src knock-down. (**C**) Expression analysis of STAT3 target genes after specific knock-down of Src.

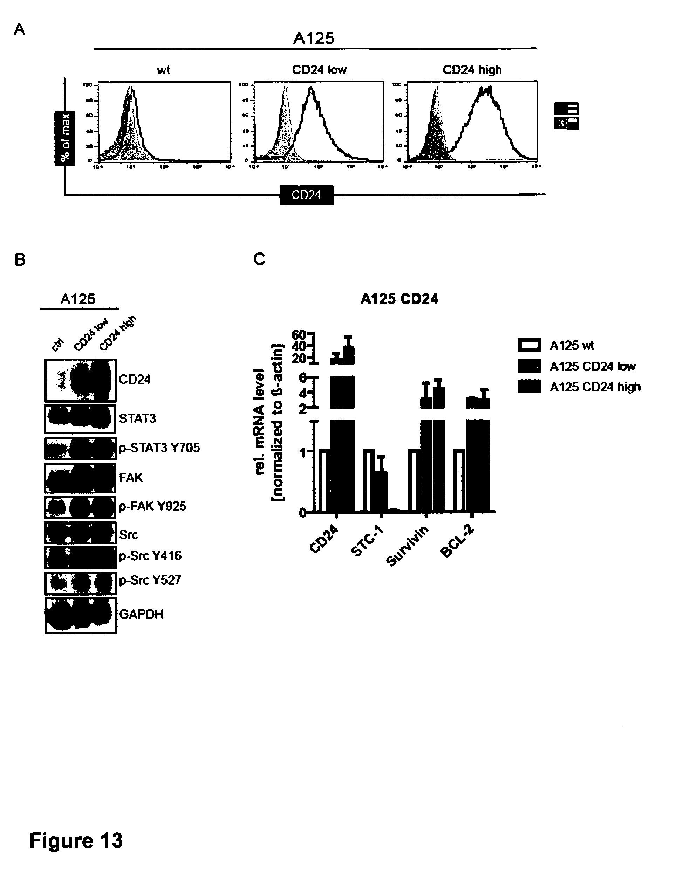

**Fig. 13** shows an Effect of CD24 overexpression on phosphorylation and gene regulation (**A**) Cytofluorographic analysis for CD24 expression in A125, A125-CD24low and A125-CD24high expressing cells. (B) Western blot analysis of cell lysates with the indicated phospho-specific antibodies. (C) Regulation of selected STAT-3-dependent target genes.

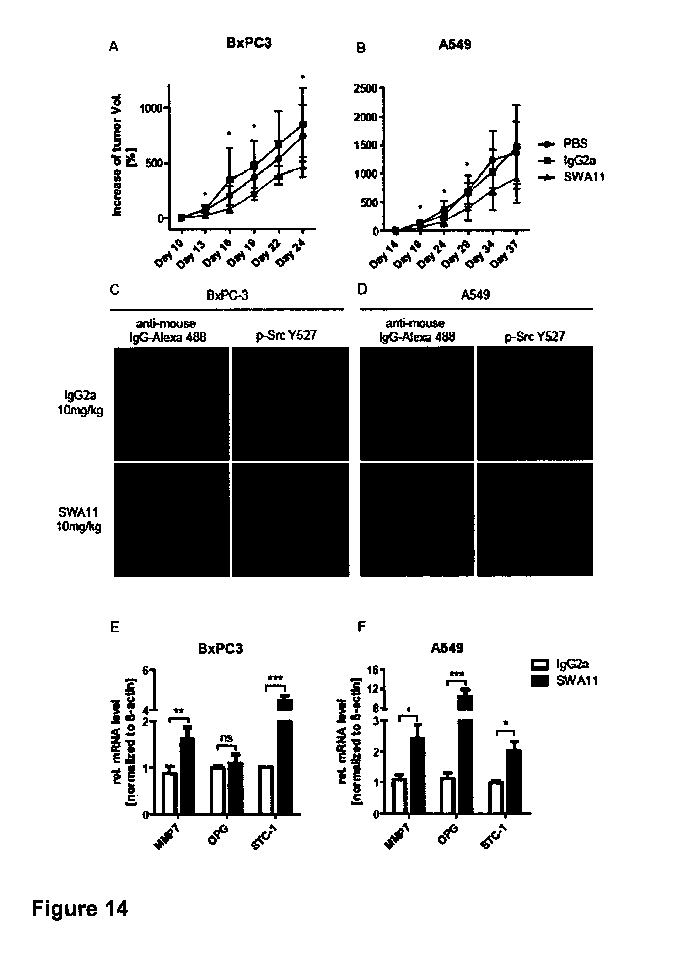

**Fig. 14** illustrates that antibodies against CD24 affect phosphorylation of Src in vivo and have a therapeutic effect (**A**) Subcutaneous tumor growth of A549 lung cancer cells in Nod/beige mice treated with either PBS, isotype control or SWA11 mAb. Antibodies (10 mg/kg) were given every 5 days with 5 injections in total. Tumor growth was measured. Animals were sacrificed and the isolated tumor was used for further analysis. (**B**) BxPC3 tumor cells were injected s.c. into NOD/SCID mice and treated with SWA11 mAb (10 mg/kg) as above. Animals were sacrificed and the isolated tumor was used for further analysis. (**C and D**) Immunofluorescent analysis of Src-phosphorylation (Y-527) in SWA11 versus isotype-control treated animals. Phospho-Src A-527 staining is increased in tumor areas where mAb SWA11 is bound. (**E and F**) MRNAs were extracted from treated tumors and analyzed by RT-PCR for the indicated STAT3-dependent genes.

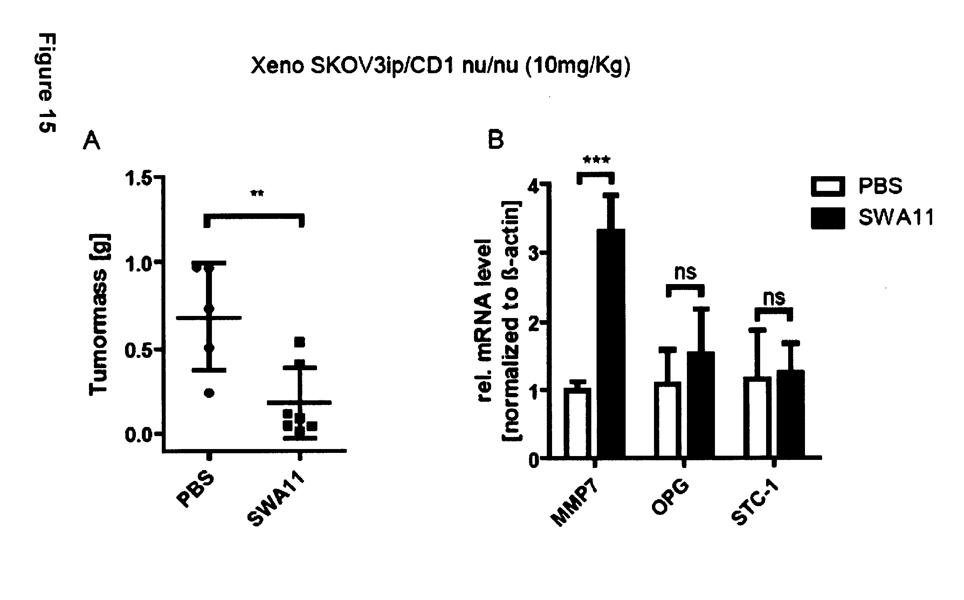

**Fig. 15** (**A**) Intraperitoneal tumor growth of SKOV3ip ovarian cancer cells in CD1 mice treated with either PBS, isotype control or SWA11 mAb. Antibodies (10 mg/kg) were given every 5 days with 7 injections in total. Animals were sacrificed, the tumor mass was determined and used for further analysis. (**B**) mRNAs were extracted from the isolated tumors and analyzed by RT-PCR for the indicated STAT3-dependent genes. c-Src Y527 staining was undetectable in both SWA11 and isotype control group.

### SUMMARY OF THE INVENTION

Amongst others, the invention relates to a method for determining whether a subject is susceptible to the treatment of a neoplastic condition with a CD24 inhibitor as defined in the claims, as well as a method for determining whether a subject suffering from a neoplastic condition has increased chances of a beneficial outcome as defined in the claims.

In addition, the present invention relates to CD24 inhibitor for use in a method for treating a neoplastic condition in a subject, wherein said subject has been identified by means of a determining method of the invention. Similarly, the invention relates to a CD24 inhibitor for use in a method for treating a neoplastic condition in a subject, comprising i) treating a sample of said subject with a CD24 inhibitor, ii) determining certain expression and/or phosphorylation levels in the sample and iii) administering a CD24 inhibitor to said subject provided that said treated sample shows respective levels.

### DETAILED DESCRIPTION OF THE INVENTION

The present inventors have performed detailed studies on CD24, CD24 signaling and effects of inhibiting CD24 to surprisingly arrive at the subject-matter of the present invention.

In a first group of experiments, the present inventors have studied the mechanism of CD24-dependent cell invasion using transient (e.g. siRNA-mediated) CD24 knock-down or (stable) CD24 overexpression in (human) cancer cell lines and surprisingly show that CD24 depletion reduced tumor cell invasion and up-regulated expression of TFPI-2 (Tissue Factor Pathway Inhibitor 2), a potent inhibitor of extracellular matrix (ECM) degradation that can block metastases formation and tumor cell invasion. Overexpression of CD24 in A125 cells resulted in reduced TFPI-2 expression and enhanced invasion. Moreover, they provide evidence that the activity of c-Src is reduced upon CD24 knock-down and that silencing of c-Src, similar to CD24, was able to enhance TFPI-2 expression and reduce tumor cell invasion. In addition, an inverse expression of CD24 and TFPI-2 was observed by immunohistochemical analysis of primary breast cancers (N=1174), where TFPI-2 expression was highest in CD24 negative samples and lowered with increasing CD24 expression. Patients with a CD24 low/TFPI-2 high phenotype showed significantly better survival compared to CD24 high/TFPI-2 low patients. These results provide evidence that TFPI-2 is as a target gene regulated by CD24, that CD24 can regulate cell invasion via TFPI-2 (a greatly reduced cell invasion was observed after CD24 knock-down or TFPI-2 overexpression) and suggest a role of c-Src in this process, as CD24 depletion altered Src-phosphorylation and Src knock-down experiments provided evidence for an important role of Src in the regulation of TFPI-2.

In a second group of experiments, the present inventors have further analyzed the functional consequences of CD24 knock-down in human cancer cell lines and show that CD24 depletion reduced cell proliferation and adhesion and up-regulated expression of various genes some of which were identified as STAT3 target genes. They provide evidence that CD24 knockdown /depletion affected (i.e. reduces) STAT3 and FAK phosphorylation, and affected STAT3-depedent transcriptional activity and gene expression. Furthermore, diminished STAT3 activity was confirmed by reporter assays and it was shown that altered activity of c-Src after CD24 knock-down is responsible for the reduced STAT3 activity and that the silencing of c-Src, similar to CD24, altered expression of prototype STAT3 target genes. Moreover, it was found that an antibody (mAb) to CD24 was effective in reducing tumor growth of human cancer cells, i.e. A549 lung cancer and BxPC3 pancreatic cancer xenografts in (immune-deficient) mice. Antibody treatment affected the level of Src-activity in the tumor and altered expression of STAT3 target genes. These results provide evidence that CD24 regulates STAT3 and FAK activity and suggest an important role of c-Src in this process and that the targeting of CD24 by antibodies likely represents a route for various tumor therapies.

These findings show for the first time that CD24 can regulate the expression of TFPI-2. They support an important link between CD24 and c-Src or possibly other members of the Src-kinase family and are considered to suggest that the depletion of CD24 in tumors offers a new the possibility to interfere with tumor progression. In addition, these results provide first evidence that siRNA to CD24 and/or binding of a CD24 specific mAb can cause similar functional effects that are beneficial for therapy. Moreover, these results demonstrate that CD24 can affect the c-Src activity and subsequently the Src-mediated phosphorylation of STAT3 and FAK. The constitutive STAT3 activation induces cancer-promoting activities involved in cell proliferation, metastasis or resistance to apoptosis.

Taken together, these novel and complex insights into CD24 function are considered to significantly improve actual medical uses and diagnostic applications such as those defined in the claims. Especially, these findings enable the determination whether a given tumor patient is susceptible to a tumor treatment using a CD24 inhibitor or whether a given tumor patient has increased chances of a beneficial outcome.

In a first aspect, the present invention relates to a method for determining whether a subject is susceptible to the treatment of a neoplastic condition with a CD24 inhibitor comprising the step(s) of i) treating a sample of said subject, particularly of a neoplasm of said subject with a CD24 inhibitor; and iii) determining a) the level of expression of STAT3, b) the level of phosphorylation of STAT3, c) the level of phosphorylation of FAK, d) the level of phosphorylation of src at Y416, e) the level of phosphorylation of src at Y527, f) the level of expression of OPG, g) the level of expression of STC-1, h) the level of expression of MMP-7, and/or j) the level of expression of TFPI-2; wherein a) a decreased level of expression of STAT3, b) a decreased level of phosphorylation of STAT3, c) a decreased level of phosphorylation of FAK, d) a decreased level of phosphorylation of src at Y416, e) an increased level of phosphorylation of src at Y527, f) an increased level of expression of OPG, g) an increased level of expression of STC-1, h) an increased level of expression of MMP-7, and/or j) an increased level of expression of TFPI-2 in said sample is indicative of the subject being susceptible to said treatment.

Step (i) may further comprise the step of obtaining said sample from the subject.

CD24 inhibitors and CD24 antagonists, respectively, are well known to the skilled person. As used herein, a "CD24 inhibitor" is an agent that is capable of counteracting CD24 activity. Said inhibitors may act on various cellular levels reaching from the post-translational level (e.g. by binding to CD24) over post-transcriptional gene silencing (such as by an inhibitory RNA) to inhibition of transcription. These include, but are not limited to antibodies and siRNA.

Accordingly, in particularly preferred embodiments, CD24 inhibitors of the invention are selected from the group consisting of i) an anti-CD24 antibody and/or ii) a CD24 siRNA.

Anti-CD24 antibodies including monoclonal anti-CD24 antibodies are well known in the art and may easily be obtained by the skilled person. As used herein, by an "anti-CD24 antibody" is particularly meant a protein of the immunoglobulin family that is capable of specifically combining, interacting or otherwise associating with an antigen, i.e. CD24. Preferably, an anti-CD24 antibody has the ability of specifically binding to CD24. In the context of the present invention, the term "anti-CD24 antibody" is considered to also relate to antibody fragments including for example Fv, Fab, Fab' and F(ab')2 fragments, all of which may be prepared by standard methods. In addition, the term "anti-CD24 antibody" may also refer to a recombinant form of anti-CD24 antibody derived molecular species including stabilized Fv fragments (e.g. scFv or dsFv, or multimers thereof), or to so-called minibodies or dABs.

In some embodiments, the antibody is a polyclonal antibody. In preferred embodiments, the antibody is a monoclonal antibody. In certain preferred embodiments, the anti-CD4 antibody is selected from antibodies recognizing the LAP sequence in CD24. Such antibodies binding to a leucine-alanine-proline motif are well-known by the skilled person (cf. e.g. Weber et al., 1993). They include, but are not limited to antibodies selected from the group consisting of SWA11, ML5, ALB9, SWA21, SWA22, and OKB2. A particularly preferred anti-CD4 antibody is SWA11. Another particularly preferred anti-CD4 antibody is ML5. Another particularly preferred anti-CD24 antibody is ALB9. Accordingly, in some embodiments, the anti-CD24 antibody as used herein is selected from the group consisting of SWA11, ML5, ALB9, and other antibodies recognizing the LAP sequence in CD24.

Also CD24 siRNAs are well-known to the skilled person and may easily be obtained by the skilled person. Non-limiting examples of CD24 siRNAs that may be used in accordance with the invention include those employed in the Examples herein.

The sample to be obtained in accordance with aspects of the invention is not particularly limited. In preferred embodiments, however, the sample is selected from tumor tissue, peripheral blood or cerebrospinal fluid. In preferred embodiments, said sample is taken from tumor tissue. Methods for obtaining said sample will depend on the nature of said sample and include biopsy or simply taking the sample with the help of a syringe.

In some embodiments of the first aspect, two or more of a), b), c), d), e), f), g), h) and j) are determined, particularly three or more, such as four or more, such as five or more, such as six or more, such as seven or more, eight or more, or even all nine of those.

In case of contradictory results, which may occur, as the person skilled in the art will understand, it will be the majority of the results which will determine whether a subject is susceptible to a treatment.

In some preferred embodiments, item ii) includes determining of a), more preferably of a) and b). In some preferred embodiments, item iii) includes determining of c). In some preferred embodiments, item iii) includes determining of j). In some preferred embodiments, item iii) includes determining of d) and e). In some preferred embodiments, item iii) includes determining of f), g) and h).

In some other embodiments, item ii) does not include determining of a), more preferably determining of a) and b). In some other embodiments, item ii) does not include determining of c). In some other embodiments, item ii) does not include determining of j). In some preferred embodiments, item iii) does not include determining of d) and e). In some other embodiments, item iii) does not include determining of f), g) and h).

In some general embodiments, item ii) includes determining levels of expression only. In other general embodiments, item ii) includes determining levels of phosphorylation only.

In a preferred embodiment of the first aspect of the present invention, the method also comprises determining the level of expression of CD24. This may also include the determination whether CD24 is present at all. The determination of CD24 level can be performed either before or after the treatment wit the CD24 inhibitor. Preferably, it is performed before said treatment. Also preferred, it is contemplated herein that the determination of CD24 expression levels is not necessary, since in the absence of any CD24 expression no change in the expression or phosphorylation of the other indicated proteins is expected, which would mean that the respective subject is not susceptible to a treatment with a CD24 inhibitor as determined by the present invention.

Levels of expression can be determined by various ways, all of which are well-known to the skilled person. Exemplary ways that may be used are apparent from the disclosure herein, such as from the Examples herein. Likewise, levels of phosphorylation can be determined by various ways, all of which are well-known to the skilled person. Exemplary ways that may be used are apparent from the disclosure herein, such as from the Examples herein.

Generally herein, a decreased level of expression is understood to be a level that is decreased by at least 10%, such as at least 15%, 20%, 25%, 30%, 35%, 40%, 45%, and 50%, such as at least 55%, 60%, 65%, 70%, and 75%, such as at least 80%, 85%; 90%, 95%, 96%, 97%, 98%, or at least 99% when compared to a suitable control.

Generally herein, am increased level of expression is understood to be a level that is 100%, 125%, 150%, 200%, such as more than 300%, 400%, 500%, such as more than 600%, 700%, 800%, or more than 900% of that of a suitable control. Preferably, however, said increased level is not more that 1000% of that of a suitable control.

The same is explicitly also true as to increased and decreased levels of phosphorylation.

In a preferred embodiment of the first aspect, said increased and/or decreased level(s) is/are verified by means of a control, such as a control in the absence of a treatment with a CD24 inhibitor. Suitable controls are readily apparent to the skilled person, particularly in the light of the present disclosure.

Generally herein, the use of a control is, however, not mandatory, since the skilled person ― particularly the one in knowledge of the present invention ― will be able to determine an increased or decreased level on the basis of the respective measurement per se.

In a further preferred embodiment of the first aspect of the invention, said method further comprises the treatment of said sample with an anti-tumor agent, wherein it is determined whether said subject is susceptible to a treatment with a combination of said CD24 inhibitor and said antitumor agent.

In a second aspect, the present invention relates to a method for determining whether a subject suffering from a neoplastic condition has increased chances of a beneficial outcome, the method comprising at least two of the below step(s) of a) determining the level of expression of STAT3, b) determining the level of phosphorylation of STAT3, c) determining the level of phosphorylation of FAK, d) determining the level of phosphorylation of src at Y416, e) determining the level of phosphorylation of src at Y527, f) determining the level of expression of OPG, g) determining the level of expression of STC-1, h) determining the level of expression of MMP-7, j) determining the level of expression of TFPI-2; and/or k) determining the level of expression of CD24; in a sample of said subject, wherein a) a decreased level of expression of STAT3, b) a decreased level of phosphorylation of STAT3, c) a decreased level of phosphorylation of FAK, d) a decreased level of phosphorylation of src at Y416, e) an increased level of phosphorylation of src at Y527, f) an increased level of expression of OPG, g) an increased level of expression of STC-1, h) an increased level of expression of MMP-7, j) an increased level of expression of TFPI-2 and/or k) a decreased level of expression of CD24; is indicative of the subject having increased chances of a beneficial outcome.

As used herein, "increased chances of a beneficial outcome" are not particularly limited and may e.g. refer to increased chances of survival or prolonged survival. In other embodiments, increased chances of a beneficial outcome refer to an improvement in any symptom associated with such neoplastic condition, or in any combination thereof. This may include prolonged survival or disease free survival.

In preferred embodiments, chances of a beneficial outcome are understood to increased if they are increased when compared to an average patient ― particularly in case they are more than 125%, 150%, 200%, such as more than 300%, 400%, 500%, such as 600%, 700%, 800%, or more than 900% of the average chances of a patient.

In some preferred embodiments of the second aspect, three or more of a), b), c), d), e), f), g), h) j) and k) are included, particularly four or more, such as five or more, such as six or more, such as seven or more, eight or more, nine or more, or even all ten of those.

In some preferred embodiments, the method includes steps a), more preferably steps a) and b). In some preferred embodiments, the method includes step c). In some preferred embodiments, the method includes step j). In some preferred embodiments, the method includes steps d) and e). In some preferred embodiments, the method includes steps f), g) and h).

In some other embodiments, the method does not include step a), more preferably steps a) and b). In some other embodiments, the method does not include step c). In some other embodiments, the method does not include step j). In some preferred embodiments, the method does not include steps d) and e). In some other embodiments, the method does not include steps f), g) and h).

In some general embodiments, the method includes determining levels of expression only. In other general embodiments, the method includes determining levels of phosphorylation only.

Particularly as to the second aspect, in a general embodiment, the method includes step k). In an alternative general embodiment, the method does not include step k).

In a preferred embodiment of the second aspect, said increased and/or decreased level(s) is/are verified by means of one or more control(s). Suitable controls are readily apparent to the skilled person, particularly in the light of the present disclosure.

In certain preferred embodiments such control(s) is/are are selected from the following controls in dependence of the respective level(s) of interest: for said levels defined in items a), f), g), h), j) and k) determining the level of expression of one or more house-keeping genes; for said level defined in item b) determining the level of expression of STAT3; for said level defined in item c) determining the level of expression of FAK; and for said levels defined in items d) and e) determining the level of expression of src.

House-keeping genes including house-keeping genes suitable for the above control(s) are also very well known to the skilled person. They include, but are not limited to beta-actin.

Other suitable controls for each of a), b), c), d), e), f), g), h), j) and k) is comparison to an average level of respective samples of other subjects, particularly other subjects suffering from a neoplastic condition, but also a comparison to respective samples of subjects not suffering from a neoplastic condition.

Preferably, a method of the second aspect comprises a step of taking a sample of said subject. Preferably, the said level(s) is/are determined in said sample.

In a third aspect, the present invention relates to a CD24 inhibitor for use in a method for treating a neoplastic condition in a subject susceptible to said treatment, wherein said subject has been determined to be susceptible to said treatment by means of a method according to the first aspect of the invention. Accordingly, the preferred embodiments relating to the first aspect are also preferred with respect to the third aspect.

Preferably, said method of treating a neoplastic condition also includes the administration of an anti-tumor agent.

In a further, related aspect, the present invention also relates to a CD24 inhibitor for use in a method for treating a neoplastic condition in a subject susceptible to said treatment, wherein said method further comprises the administration of an anti-tumor agent, and wherein said subject has been determined to be susceptible to said combined treatment by means of a method as described above.

In a further, related aspect, the present invention relates to a CD24 inhibitor for use in a method for treating a subject suffering form a neoplastic condition, wherein said subject has been determined to have increased chances of a beneficial outcome by means of a method according to the second aspect of the invention.

In a fourth aspect, the present invention relates to a CD24 inhibitor for use in a method for treating a neoplastic condition a subject, said method comprising i) treating a sample of said subject, particularly of a neoplasm of said subject with a CD24 inhibitor; ii) determining a) the level of expression of STAT3, b) the level of phosphorylation of STAT3, c) the level of phosphorylation of FAK, d) the level of phosphorylation of src at Y416, e) the level of phosphorylation of src at Y527, f) the level of expression of OPG, g) the level of expression of STC-1, h) the level of expression of MMP-7, and/or j) the level of expression of TFPI-2; and iv) administering a CD24 inhibitor to said subject provided that said treated sample shows a) a decreased level of expression of STAT3, b) a decreased level of phosphorylation of STAT3, c) a decreased level of phosphorylation of FAK, d) a decreased level of phosphorylation of src at Y416, e) an increased level of phosphorylation of src at Y527, f) an increased level of expression of OPG, g) an increased level of expression of STC-1. h) an increased level of expression of MMP-7, and/or j) an increased level of expression of TFPI-2 in said sample.

All embodiments described above for the method according to first aspect of the invention also apply to said fourth aspect of the invention.

Especially, said method further comprises the treatment of said sample with an anti-tumor agent, wherein it is determined whether said subject is susceptible to a treatment with a combination of said CD24 inhibitor and said antitumor agent, and wherein said method further comprises the administration of said ant-tumor agent to said subject.

In preferred embodiments, said CD24 inhibitor i) is an anti-CD24 antibody, particularly a monoclonal anti-CD24 antibody; or ii) is a CD24 siRNA.

Generally herein, a "neoplastic condition" is not particularly limited and is used synonymously with a "tumor". Neoplastic conditions and tumors are well known to the skilled person.

Generally, in preferred embodiments of all aspects of the invention, the neoplastic condition is selected from the group consisting of astrocytoma, B-cell lymphoma, brain carcinoma, breast adenocarcinoma, breast carcinoma, Burkitt's B-cell lymphoma, cervical adenocarcinoma, colon adenocarcinoma, colorectal adenocarcinoma, colorectal carcinoma, glioblastoma, hepatocellular carcinoma, hepatoma and pancreatic carcinoma, leukaemia, large cell lung cancer, lung adenocarcinoma, lung carcinoma, lung mesothelioma, lung squamous cell carcinoma, melanoma, neuroblastoma, non small cell and small cell lung cancer (NSCLC and SCLC), oesophageal cancer, ovarian adenocarcinoma, pancreatic adenocarcinoma, pancreatic carcinoma, prostate adenocarcinoma, prostate carcinoma, rectal adenocarcinoma, renal cell adenocarcinoma, small cell lung cancer (SCLC), T-cell lymphoma, thyroid cancer, and uterine cancer.

Even more preferably, it is selected from the group consisting of brain carcinoma, breast adenocarcinoma, breast carcinoma, large cell lung cancer, lung adenocarcinoma, lung carcinoma, lung mesothelioma, lung squamous cell carcinoma, non small cell and small cell lung cancer, and ovarian adenocarcinoma.

In some preferred embodiments, the neoplastic condition is one that is associated with an increased expression of CD24, including those described herein. In some preferred embodiments, the neoplastic condition is one that has been tested directly or indirectly in the Examples herein.

Preferred examples are pancreas, breast, ovarian and lung cancer.

In case that an anti-tumor agent is used in the context of the present invention, the following applies.

If an anti-tumor agent is applied or administered in combination with a CD24 inhibitor, this means that the anti-tumor agent may be applied or administered before, together or after said CD24 inhibitor.

Anti-tumor agents are well-known to the skilled person. As used herein, the term "a further anti-tumor agent" is intended to refer to one or more, but preferably one anti-tumor agent that is not a CD24 inhibitor in the sense of the present invention. Accordingly, as used herein, said term does not include any of the preferred CD24 inhibitors described herein. To simplify matters, said term may be used equivalently with the term "an anti-tumor agent". In other words, for the purposes of the present invention, the term "an anti-tumor agent" as used herein preferably refers to "a further anti-tumor agent" as defined above.

In some preferred embodiments, said further anti-tumor agent is selected from the group consisting of a chemotherapeutic agent and a radiotherapeutic agent, both of which are well-known to the skilled person (cf. e.g. Remington's Pharmaceutical Sciences, 5th ed., chapter 33, in particular pages 624 to 652).

Preferably, said chemotherapeutic agent is selected from the group consisting of an anti-metabolite, an alkylating agent, a plant alkaloid, a topoisomerase inhibitor, and a cytotoxic /antineoplastic antibiotic. Also suitable are natural source derivatives as well as any of asparaginase, mitotane and C2 ceramide.

Examples of antimetabolites that can be used in the invention include but are not limited to fluorodeoxyuridine, cladribine, cytarabine, floxuridine, fludarabine, flurouracil such as 5-fluorouracil (5FU), gemcitabine, hydroxyurea, mercaptopurine, methotrexate, and thioguanine. In some preferred embodiments, said anti-metabolite is a nucleoside analog, especially selected from gemcitabine and fluorouracil. Examples of alkylating agents that can be used in the invention include but are not limited to busulfan, caroplatin, carmustine, chlorambucil, cisplatin, cyclophosphamide (i.e., cytoxan), dacarbazine, ifosfamide, lomustine, mecholarethamine, melphalan, procarbazine, streptozocin, and thiotepa. Examples of antineoplastic antibiotics that can be used in the invention include but are not limited to include bleomycin, dactinomycin, daunorubicin, doxorubicin, idarubicin, mitomycin (e.g., mitomycin C), mitoxantrone, pentostatin, and plicamycin. Examples of natural source derivatives include docetaxel, etoposide, irinotecan, taxanes (e.g. paclitaxel), teniposide, topotecan, vinblastine, vincristine, vinorelbine, prednisone, and tamoxifen.

Preferably, as used herein, said radiotherapeutic agent (which may also be referred to as "radiotherapy") includes every radiation therapy which is commonly used to treat tumors cells. In preferred embodiments, said agent / therapy include γ-rays, X-rays, microwaves, UV radiation as well as the direct delivery of radio-isotopes to or next to tumor cells (brachytherapy). In preferred embodiments, said agent / therapy include agents coupled to or comprising a radioisotope.

Generally in the aspects herein, a CD24 inhibitor is preferably administered to a subject in form of a composition, such as a pharmaceutical composition comprising said inhibitor. Likewise, when "treating a sample with a CD24 inhibitor", as used herein, said CD24 inhibitor may be comprised in a composition.

The CD24 inhibitor as used herein and the anti-tumor agent are preferably used in pharmaceutical compositions. These may comprise one or more pharmaceutically acceptable carriers. Examples of pharmaceutically acceptable carriers are well-known and e.g described in standard text books.

Generally, the term "carrier" refers to a diluent, adjuvant, excipient, and/or vehicle, or any combinations thereof, that may be comprised in the compositions herein.

Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain wetting or emulsifying agents, and/or pH buffering agents. The compositions can take the form of solutions, suspensions, emulsions, tablets, pills, capsules, powders, sustained-release formulations and the like. The composition can be formulated as a suppository, with traditional binders and carriers such as triglycerides. Oral formulation may include standard carriers such as pharmaceutical grades of mannitol, lactose, starch, magnesium stearate, sodium saccharine, cellulose, magnesium carbonate, etc.

The compositions herein preferably contain a therapeutically effective amount of the therapeutic, preferably in purified form, particularly together with a suitable amount of carrier so as to provide the form for proper administration to the patient. The formulation should suit the mode of administration. Throughout the invention, the term "effective amount" means that a given molecule or compound is administered in an amount sufficient to obtain a desired therapeutic effect. In case that two compounds are administered in a therapeutic effective amount, this includes that one or each of the compounds is administered in a subtherapeutic amount, i.e. that the amount of each compound on its own is not sufficient to provide a therapeutic effect, but that the combination of the compounds results in the desired therapeutic effect. However, it is also included within the present invention that each of the compounds on its own is administered in a therapeutically effective amount.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the therapeutic of the invention, which will be effective in the treatment of a particular disorder or condition, will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis; construction of a therapeutic nucleic acid as part of a retroviral or other vector, etc.. Methods of introduction include but are not limited to intradermal, intramuscular, intraperitoneal, intravenous, subcutaneous, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, nonporous, or gelatinous material, including membranes, such as sialastic membranes, or fibers. In one embodiment, administration can be by direct injection at the site (or former site) of a malignant tumor or neoplastic or pre-neoplastic tissue.

In the following, those findings of the present inventors which are described in detail in the Examples below will be discussed in further detail to emphasize their significance.

That is, in the first group of studies, CD24 and TFPI-2 have been independently found to modulate the invasion of human tumor cells. The present inventors provide evidence that in tumor cell lines an inverse correlation exists between CD24 and TFPI-2 and demonstrate that i) CD24 knock-down abrogates cell invasion and up-regulates TFPI-2; ii) augmented TFPI-2 expression appears to be responsible for the inhibition of invasion; iii) the regulation of TFPI-2 and cellular invasion seems to depend on the cross-talk of CD24 with the lipid-raft associated kinase Src. These findings combine independent observations and shed light on a novel mechanism of CD24-dependent regulation of tumor cell invasion. In detail, TFPI-2 was identified by genome wide expression profiling after CD24 knock-down in SKOV3ip cells. The strong up-regulation was confirmed by RT-PCR at the mRNA and by Western blotting at the protein level ― and the up-regulation of TFPI-2 mRNA was not only detected in SKOV3ip but also in HS683 and A549 cells. Concomitantly, in all three cell lines a significantly reduced cell invasion that was noticed which was not seen in SNB 19 cells having no detectable levels of TFPI-2. In contrast, the overexpression of TFPI-2 in these cells restored TFPI-2 expression and inhibited cell invasion. These results extended prior results reported in a paper published by Konduri et al. (Konduri et al., 2001) and led to the conclusion that TFPI-2 may act as a mediator of CD24-dependent cell invasion. This was supported by experiments in A125 lung adenocarcinoma cells showing that the stable overexpression of CD24 suppressed TFPI-2 and augmented invasion. Importantly, the present findings establish a link between CD24 and TFPI-2, a proteinase inhibitor known for its important role in the inhibition of tumor cell invasiveness, neoplastic growth, and metastases formation (Sierko et al., 2007). Further support for a functional link between CD24 and TFPI-2 was obtained by immunohistochemical staining of human breast tumor sections. CD24 high staining went along with a diminished expression of TFPI-2. In contrast, a CD24 low expression was usually linked to TFPI-2 high staining and these tumors showed a "knock-out" phenotype similar to the cell lines. These findings are consistent with previous studies indicating that CD24 high and TFPI-2 low expression are independent markers for a bad prognosis. Tumor cell invasion can be influenced by a variety of factors, including integrins, proteolytic activity, proteolytic inhibitors or the ability to alter cell shape and generate contraction. Here, the knock-down of CD24 did not lead to changes in p1-integrin or MMP-2/ MMP -9 expression levels. Interestingly, it was previously described that CD24 overexpression augmented contractile forces necessary for cell invasion and this was blocked by knockdown of p1-integrin or by myosin light chain and Src inhibitors (Mierke et al., 2011). These results suggested that CD24, in addition to the observed effects on TFPI-2 levels as described in the present study, may enhance cell invasion through increased generation or transmission of contractile forces.

As an aside, a recent study has shown that CD24 can modulate cell invasion and motility in still another way. In pancreatic cancer cells the suppression of CD24 by siRNA caused enhanced cell motility and invasion in vitro and fostered retroperitoneal and hepatic metastasis when injected into nude mice (Taniuchi et al., 2011). Here, the formation of a complex between CD24 in cytoplasmic vesicles with G3BP in stress granules, which are formed in response to various stress signals and are involved in mRNA stability, was reported (Taniuchi et al., 2011). G3BP can both bind and cleave and thereby inactivate specific mRNAs (Kedersha et al., 2002). More specifically, the CD24/G3BP was found to affect the stability and abundance of BART, a cytoplasmic binding partner of the small G-protein ARL2 that regulates different microtubule -dependent processes (Zhou et al., 2006). In the presence of CD24 the cleavage of BART mRNA by G3BP was blocked whereas both BART and CD24 siRNA depletion resulted in enhanced cell motility and invasion (Taniuchi et al., 2011). This work suggested a novel role of CD24 as a post-transcriptional regulator of G3PB and thereby of mRNA stability. These important new findings await confirmation in other experimental systems.

The present inventors further observed that CD24 knock-down altered the activation status of Src with a decrease of Phospho-src Y416 (active Src) and an increase of Phospho-src Y527 (inactive Src). The Src N-terminal region contains a myristic acid moiety essential for the localization at the inner surface of the membrane. Some association of the GPI-anchored CD24 and members of the Src-family of kinases such as p561ck, p56hck, and p541yn in immune cells (Sammar et al., 1997) and fgr in lung cancer cells (Zarn et al., 1996) most likely due to their association in lipid rafts, had been suggested. The activity of c-Src is regulated by intramolecular interactions that depend on the equilibrium between tyrosine phosphorylation and dephosphorylation, i.e. the activity of kinases and phosphatases (Yeatman et al., 2004) (Guarino et al., 2010). Interestingly, the lipid raft-specific knock-down of c-Src inhibits cell adhesion and cell cycle progression in breast cancer cells (Hitosugi et al., 2007). The present inventors tested whether changes in c-Src activity might are involved in altered TFPI-2 expression. Indeed, specific knock-down of Src reduced cell invasion was observed (see also Koppikar et al., 2008). The knock-down of c-Src in SKOV3ip or A549 cells augmented the amount of TFPI-2 deposited in the ECM and augmented the TFPI-2 mRNA levels in both cell lines. Since c-Src activity can trigger a variety of signaling pathways including Ras/MAPK, Pi3K/Akt or STAT3 it remains to be established which particular signaling pathway is involved in TFPI-2 regulation. It can, however, be concluded that c-Src activity either at the transcriptional or posttranscriptional level is involved in the regulation of TFPI-2 expression. Other supporting evidence for an important role of Src as a CD24-mediator comes from a recent study showing that CD24 interacts with and promotes the activity of c-Src within lipid rafts in breast cancer cells and thereby augments integrin-dependent adhesion by increased phosphorylation of FAK and paxillin (Baumann et al., 2011). The TFPI-2 gene is considered a tumor suppressor gene as its down-regulation or loss is associated with increasing malignancy (Sierko et al., 2007). Previous studies have shown that TFPI-2 suppression is mediated by promoter methylation in highly invasive breast cancer cells (Guo et al., 2007). Other studies have implicated the ERK/MAPK pathway as a regulator of the TFPI-2 promoter (Kast et al., 2003). TFPI-2 has been also identified as a target gene of STAT3 signaling (Dauer et al., 2005). The results obtained in the present studies have, however, revealed that the transient knock-down of CD24 does not alter the methylation pattern of the TFPI-2 promoter in SKOV3ip cells (data not shown). Moreover, no evidence was obtained that STAT3 knock-down in SKOV3ip or A549 cells affected the TFPI-2 expression levels ruling out the possibility that a Src-STAT3 axis is involved in the regulation of TFPI-2 (data not shown). Additional experiments are envisaged to further elucidate the signaling pathway and the functional role of c-Src in the regulation of TFPI-2.

In detail, in the second group of studies, using human cancer cells as a model, it was investigated how CD24 affects cellular functions and it was, among others, found that i) CD24 depletion affects FAK and STAT3 phosphorylation and STAT3-transcriptional activity; ii) CD24 knock-down alters the expression of STAT3 dependent genes; iii) the CD24 mediated regulation of STAT3 genes requires Src activity; iv) treatment of mice bearing A459 or BxPC3 tumors with mAb directed against CD24 alters Src activity and STAT3 gene expression *in vivo.* These findings suggest an unexpected and novel role of CD24 in the regulation of STAT3 activity that may explain the association of CD24 with a poor prognosis in cancer. In even more detail, the present inventors initially observed that the CD24 knock-down affected cell proliferation in three out of four cell lines tested and this was paralleled with the induction of apoptosis. In addition, there was also a diminished adhesion of two out of four cell lines towards the α4-integrin substrate FN-40 whereas the binding to laminin was not affected. These effects of CD24 on functional characteristics of tumor cells are in line with previous publications showing a role of CD24 e.g. in cell proliferation, adhesion and invasion (Ahmed et al., 2009; Baumann et al., 2005; Baumann et al., 2011; Fukushima et al., 2007; Mierke et al., 2011; Runz et al., 2008; Sagiv et al., 2008; Schabath et al., 2006; Senner et al., 1999; Smith et al., 2006; Wang et al., 2010). Importantly, the present inventors also observed a decreased phosphorylation of FAK-Y925 after CD24 knockdown and consistently an enhanced phosphorylation of the same site in A125―CD24 high cells. This site in FAK is known to be phosphorylated by Src-kinases and the lack of Src kinase-dependent phosphorylation at this site is associated with impaired adhesion turnover (Brunton et al., 2005). Thus, these results provide first evidence for an actual role of Src-kinases in CD24-mediated effects (see also Baumann et al., 2011). The results presented in the present studies, however, extend these initial observations and allow unexpected medical applications and the like.

To identify additional changes induced by CD24, the present inventors carried out genome wide expression profiling after CD24 knock-down in SKOV3ip cells. Focusing on genes relevant in cancer biology (including OPG, MMP7, STC-1 and STAT3) their strong regulation was confirmed by q-PCR. The changes in the expression of the selected genes were also found in the other cell lines investigated. Importantly, STAT3 was down-regulated by CD24 depletion and this was observed with five different CD24-specific siRNAs ruling out any off-target effect. The present inventors also noticed consistently a decrease of STAT3 protein expression. In addition and/or as a result of STAT3 reduction, a significant decrease in p-STAT3 Y705 in the cell lines employed was noticed and, subsequently, a loss of transcriptional activity in a STAT3-driven luciferase reporter assay. Typical STAT3-dependent genes such as cyclin D1, survivin and MCL-1 were decreased in expression, and the CD24-target genes OPG, MMP7 and STC-1 were also indentified as STAT3-dependent. Importantly, the knock-down of either CD24 as well as c-Src resulted in similar changes in the expression of STAT3 target genes. When CD24 was overexpressed in A125 cells, a strong increase in p-STAT3 Y705 and p-Src Y416 phosphorylation in A125-CD24high cells was observed. It is noteworthy that there was no increase of STAT3 protein upon CD24 overexpression in the latter cells. Nevertheless, the presence of CD24 led to the increased expression of STAT3-target genes. Thus, these data demonstrate that CD24 affects the transcriptional activity of STAT3 through Src activation and alters the expression of STAT3-dependent genes.

A recent study in hepatocellular carcinoma has shown that CD24 is up-regulated in putative tumor-initiating cells that were enriched for by their resistance to chemotherapy (Lee et al., 2011). The knock-down of CD24 suppressed the stem/progenitor cell characteristics suggesting that CD24 is a functional marker for tumor-initiating cells in the liver. Importantly, the stemness-associated gene NANOG was identified as a downstream target gene of CD24 and the regulation of NANOG expression was through STAT3 phosphorylation at Y705 (Lee et al., 2011). Manipulation of CD24 expression consistently altered the activate form of Src (pY416) and thus, the authors concluded that CD24 potentially phosphorylated STAT3 through Src but not JAK. These published results are in support of the data of the present invention. The classical activation pathway of STAT3 is via upstream kinases such as JAK2 that links STAT3 signaling to cytokine receptors (Yu et al., 2009). A recent study has proposed that the STAT3 activation is required for the growth of CD44+CD24- stem cell-like breast cancer cells (Marotta et al., 2011). This phenotype appears to be enriched in basal-like breast tumors (Honeth et al., 2008) and was previously proposed to identify breast cancer stem cells (Al-Hajj et al., 2003). Using primary tumor material and breast cancer cell lines the authors identified the IL6/Jak2/STAT3 pathway as being crucial for the growth of basal-like breast cancer cells (Marotta et al., 2011). When analyzing breast tumor tissues sections by triple fluorescence analysis of CD24, CD44 and pSTAT3 it became clear that positive staining for phospho-STAT3 was not restricted to the CD44+CD24- cells but resided also in >50% of CD44+CD24+ cells (Marotta et al., 2011). Thus, in the light of the present data it is possible that CD24-Src signaling contributed to STAT3 phosphorylation and hence, the IL-6/Jak2 pathway is not the only driver of STAT3 activation.

Another recent study has shown that CD24 is a promising therapeutic target for anti-metastatic therapy of bladder cancer (Overdevest et al., 2011). It was first demonstrated by comparing paired primary and metastatic human bladder cancer samples that CD24 expression was increased in the metastases. Using a model of human urothelial cancer cells transplanted into immunocompromised mice and therapy with anti-CD24 mAb ALB9 a significantly reduced tumor growth and prolonged survival was observed (Overdevest et al., 2011). Thus, CD24 seems to be an important cellular factor for metastatic progression in bladder cancer and may be promising therapeutic target for anti-metastatic therapy. In the present invention, these published data were confirmed and extended by demonstrating that the mAb SWA11 directed against CD24 can inhibit the growth of BxPC3 pancreatic cancer and A549 lung carcinoma cells *in vivo.* The present inventors provide evidence by immunohistochemistry that the mAb directed against CD24 reaches the tumor site and increases c-Src Y527 (inactive Src) expression. Subsequent analysis of tumor tissue by q-PCR revealed that the mAb to CD24 interfered with the expression of STAT3 dependent genes.

Taken together, the present findings are considered to allow the diagnostic, therapeutic and other applications described hereinabove and in the claims.

### EXAMPLES

The following examples are meant to further illustrate, but not limit, the invention. The examples comprise technical features, and it will be appreciated that the invention also relates to any combinations of the technical features presented in this exemplifying section.

### Example 1: Materials and Methods

### Cells

The tumor cell lines A549 (lung adenocarcinoma), SKOV3ip (ovarian carcinoma) HS683 and SNB19 (glioblastoma) were either described before (SKOV3ip, (Wolterink et al., 2010)) or obtained from the tumor bank of the German Cancer Research Center. The lung adenocarcinoma cell line A125 stably transfected with CD24 (and selected by FACS sorting) / used for stable transfection of CD24 was described before (Mierke et al., 2011; Runz et al., 2008). SKOV3, SKOV3ip, HS683 and SNB19 cells were cultured in Dulbecco's modified Eagle's medium (DMEM) supplemented with 10% fetal bovine serum (FBS). A125 (stably overexpressing CD24 (Ala or Val form)) and A549 cells were maintained in RPMI 1640 containing 10% FCS.

### Chemicals and antibodies

The mAb SWA11 to human CD24 was described before (Jackson et al., 1992; Schabath et al., 2006). The antibodies against Src, phospho-Src (Y416, Y527), STAT3 and phospho-STAT3 (Y705) were obtained from Cell Signaling (Heidelberg, Germany). The mAb specific for GAPDH was from Santa Cruz Biotech (Heidelberg, Germany). TFPI-2 pAb was kindly provided by W. Kisiel, (Albuquerque, NM, USA) and has been described before (Konduri et al., 2001). Beta 1 integrin specific mAb was obtained from BD Bioscience (Heidelberg, Germany). Secondary antibodies coupled to / conjugated with either peroxidase or phycoerythrin were obtained from Dianova (Hamburg, Germany).

### siRNAs and siRNA mediated knock-down

The siRNA for CD24 was described before (Schabath et al., 2006). Additional CD24 specific oligos / CD24-targeting siRNAs were obtained from MWG (Ebersberg, Germany). siRNAs targeting STAT3, TFPI-2, and Src as well as (non-targeting) GFP-specific siRNA for controls were also obtained from MWG (Ebersberg, Germany). Sequences for siRNAs, such as siSTAT3 and siSrc, were designed using the "Ambion siRNA target finder" online-tool by Applied Biosystems (Darmstadt, Germany). Transfection of siRNAs was carried out using Oligofectamine™ (Invitrogen, Darmstadt, Germany) following the manufactures protocol.

### RNA microarray analysis

The expression analysis of SKOV3ip cells transiently transfected with siRNAs to CD24 or non targeting GFP-specific siRNA using the Illumina human Sentrix-6 v3 BeadChip was performed by the expression profiling core facility (Dr. Bernhard Korn) of the German Cancer Research Center, Heidelberg. The detailed procedure has been described before (Wolterink et al., 2010). Two independent biological replicates were analyzed. Genes with fold change of < -1.5 or ≥ 1.5 were regarded as regulated and the gene expression of selected candidates was verified using qRT-PCR.

### Quantitative real-time PCR

q(RT)-PCR was performed as described before (Riedle et al., 2009). Briefly, total RNA from cells was isolated (e.g. using the RNAeasy Mini Kit (Quiagen, Hilden, Germany)). For quantitative real-time PCR (qPCR / qRT-PCR), cDNA was and transcribed using the ReverseAid H Minus First Strand cDNA Synthesis Kit (Fermentas, St.Leon-Rot, Germany), purified on Microspin G-50 columns (GE Healthcare, Freiburg, Germany) and quantified by a NanoDrop Spectrophotometer (ND-2000; Thermo Scientific). qPCR primers were designed by using the "Primer Quest" (online) programme (IDT, Munich, Germany) and were produced by MWG Eurofines (Ebersberg, Germany). As an internal standard β -actin was used.

### Luciferase assay

This has been described in detail elsewhere (Pfeifer et al., 2010). Briefly, cells were seeded into 96-well plates and subjected to siRNA transfection as described above. The STAT3-specific or control reporter luciferase-constructs (Cignal™ STAT3 Reporter Assay Kit, SABioscience, Frederick, MD, USA) were transfected 24h after siRNA application using SureFECT™ (SABioscience, Frederick, MD, USA) transfection. Cell lysis and luciferase measurement was carried out as described (Pfeifer et al., 2010). The experiment was carried out in triplicates.

### Apoptosis assay

The apoptosis assay using annexin-V and propidium iodide (PI) staining has been described elsewhere (Stoeck et al., 2007).

### MTT assay

This assay was described in detail before (Kiefel et al., 2010).

### Fluorescence-activated cell sorting/Flow cytometry

This technique has been described in detail elsewhere (Schabath et al., 2006). Cells were analyzed with FACS Canto II (Becton Dickinson, Heidelberg, Germany). For data analysis, FlowJo software (Ashland, OR) was used.

### Cell lysis and Western blot analysis

Cell pellets were solubilized in lysis buffer (20 mM Tris/HCI (pH 8.0) containing 50 mM BOG or 1% Triton X-100, 10 mM NaF, 10 mM orthovanadate, 1 mM PMSF, 1 µg/ml of each leupeptin, aprotinin and pepstatin). Alternatively, cell pellets were solubilized in lysis buffer (250 mM NaCl, 50 mM HEPES, 0.5% NP-40, 10% glycerol, 2 mM EDTA, 10 mM NaF, 1 mM Na-orthovanadate, 1 mM PMSF, 10 mg/ml of each leupeptin and aprotinin) for 30 min on ice. Lysates were cleared by centrifugation and boiled with reducing or non-reducing SDS-sample buffer. Samples were separated on SDS-PAGE gels and transferred to Immobilon membranes or to PVDF membranes (Millipore, Schwalbach, Germany), respectively, using semi-dry blotting. E.g. after blocking with 4% BSA in Tween20/TBS, membranes were probed with primary antibodies followed by horseradish peroxidase-conjugated secondary antibody and ECL detection (Amersham-Pharmacia / GE-Healthcare, Freiburg, Germany).

### In vivo xenograft tumor models

BxPC3 human pancreatic cancer cells or A549 lung cancer cells (5 x 10⁶ in 100 µl of PBS) were transplanted s.c. into the right flank of Nod/Scid or Scid/beige mice, respectively. SWA11 mAb treatment was initiated when tumors reached a volume of 30-80 mm³. Animals (n = 7-10 per group) received five i.p. injections of SWA11 mAb (IgG2a) at a dose of 10 mg/kg. Control animals received unspecific IgG2a or PBS. Tumor size was measured externally using a caliper. Data are presented as a relative tumor volume increase from the time of antibody administration. The SKOV3ip therapy model has been described (Wolterink et al., 2010). Animal experiments were approved by the Baden-Württemberg animal oversight committee (Regierungspräsidium Karlsruhe, Germany).

### Immunofluorescence

Double immunofluorescence was performed on 6 mm frozen tumor tissue sections as described elsewhere. Goat anti-mouse IgG conjugated to Alexa 488 or goat anti-rabbit IgG Alexa 594 were used as secondary antibodies. Tissue stainings were examined at 400x magnification using a Leica DMRB microscope. Images were captured using a SPOT Flex digital color camera (Diagnostic Instruments Inc., Sterling Heights, MI, USA) and analyzed with SPOT Advanced version 4.6 software.

### Transient overexpression of TFPI-2

pcDNA3-TFPI-2 and pcDNA3-ctrl (control) plasmids were kindly provided by W. Kisiel. Overexpression was achieved by transient transfection using JetPEI™ transfection reagent from Polyplus (Erlangen, Germany) following the manufactures protocol. After 48h cells were harvested to determine the levels of TFPI-2 mRNA or protein or used for functional experiments.

### ECM preparation and TFPI-2 analysis

Preparation of ECM in vitro has been described before (Konduri et al., 2001). Briefly, cells in equal numbers (1x10⁵/well) were seeded 48h after transfection for 48h in 6-well plates. Cells were washed with PBS and lysed by 0.5% TX-100 (v/v) in PBS. The remaining ECM was washed three times with PBS and another three times with 20 mM Tris-HCl, pH 7.4, containing 100 mM NaCl and 0.1% (v/v) Tween-20. After washing, 200µl of 2x SDS-PAGE sample buffer (Invitrogen, Darmstadt, Germany) was used to collect ECM proteins from the dish by agitating slightly for 20-30 min at RT. Cell lysate was isolated to demonstrate the equal cell number of plating.
ECM samples and corresponding cell lysates were separated using SDS-PAGE and Western blotting. Equal sample volumes were loaded on gels followed by immobilization to PVDF membranes. For detection of TFPI-2 protein an anti TFPI-2 specific polyclonal antibody was used that has been described before (Konduri et al., 2001).

### Matrigel™ invasion assay

Tumor cell invasion *in vitro* was determined in a double-filter assay as described before (Gast et al., 2008). Briefly, a Matrigel™ layer was established between a 5µm pore nitrocellulose filter (bottom) and a 12µm pore polycarbonate filter (top). The assay was carried out in triplicates. 1x10⁵ cells/ml were seeded on top of the filter-sandwich for 20h. To account for differences in cell proliferation, the same number of cells was seeded into similar sized tissue culture wells and the relative proliferation was determined by cell counting. Invasion filter-sandwiches were fixed in 2.5% glutaraldehyde/PBS for at least 1h at 37°C. Following separation of the filters, cells present in the gel or attached to the lower filter were stained with DAPI. Using a fluorescence microscope, nuclei from 5 randomly taken pictures per filter were counted by ImageJ software. Cell invasion was expressed as the mean percentage of invaded cells normalized to the proliferation control.

### Gelatine zymography

This technique has been described in detail elsewhere (Runz et al., 2007).

### Immunohistochemistry

The TMA was constructed from primary breast cancers, carcinoma in situ lesions, local recurrences and distant metastases have been previously described in detail (Theurillat et al., 2007). (Kristiansen et al., 2010b). Automated IHC staining was performed using Benchmark XT (Ventana, Tucson, AZ) as described before (Kristiansen et al., 2010a). mAb SWA11 hybridoma supernatant against CD24 was diluted 1:5. DAB (Ventana, Tucson, AZ) served as chromogen.
To detect TFPI-2 with a polyclonal pAb (final dilution 1:300), automated IHC staining of formalin-fixed, paraffin-embedded tissue was performed using the Bond™ automated immunohistochemistry and In-Situ hybridization system (Leica Microsystems, Melbourne, Australia) using the Refine 30'/30' detection system and the H2 10/95°C pretreatment protocol. Afterwards the slides were briefly counterstained with hematoxylin and aqueously mounted.

### Evaluation of Immunohistochemistry

For both Abs (SWA11 and pAb to TFPI-2) the staining intensity was scored semiquantitatively, ranging from negative to strong (0, 1+, 2+ 3+) as described before (Kristiansen et al., 2010a). For statistical analysis, the data sets were dichotomized (CD24: 0-2+ (low) vs. 3+ (high); TFPI-2: 0-1+ (low) vs. 2-3+ (high)).

### Statistical analysis

Data are presented as the mean ± SD. Student's *t*-test was used to evaluate the difference between groups. P < 0.05 was considered statistically significant. P-values in the Figures are indicated as follows: *<0.05, **<0.01 ***<0.001. Alternatively, P-values in the Figures are written as numbers (cf. e.g. Fig. 6D). For IHC evaluation the statistics were calculated with SPSS V16. Spearman rank correlation was used to compare immunoreactivity. Univariate survival statistics were calculated according to Kaplan Meier with Log rank test.

### Example 2: CD24 knock-down affects invasion of cancer cell lines

The role of CD24 in cell invasion was investigated using transient knock-down of CD24 in tumor cell lines, i.e. SKOV3ip ovarian carcinoma, A549 lung carcinoma, and HS683 glioblastoma cells. Five siRNAs directed against CD24 were tested, and the oligonucleotide with the highest knock-down efficacy was selected for further studies. The efficacy of CD24 knock-down was tested by FACS analysis for cell-surface CD24 (Fig. 1A) and Western blot analysis of cell lysate (Fig. 1B) and was found to be between 60-98%. When CD24 depleted cells were tested in a matrigel invasion assay, a significantly reduced cell invasion in all cell lines was observed- (Fig. 1C). Since the levels of pl-integrin expression are critical for cell invasion into matrigel, FACS analysis was carried out. In some cell lines a small increase in pl-integrin expression after CD24 knock-down was noticed (Fig. 1A). These results suggest that a role of CD24 in the regulation of tumor cell invasion was most likely independent of pl-integrin expression levels.

### Example 3: CD24 depletion augments TFPI-2 expression

In colorectal and pancreatic carcinoma cells CD24 was shown to control gene expression by so far unknown mechanisms (Sagiv et al., 2008). To verify these observations in SKOV3ip cells, genome wide expression analysis were used to identify genes affected by CD24 knock-down compared to irrelevant GFP knock-down. A total of approx. 200 genes were indentified to be regulated more than 1.5 fold. Based on its functional importance for cell invasion, focus was put on the tissue factor pathway inhibitor-2 (TFPI-2) and a more detailed analysis was carried out. Indeed, the transient depletion of CD24 by siRNA led to a significant up-regulation of TFPI-2 not only in SKOV3ip cells but also in A549 and HS683 cells (Fig. 2A). TFPI-2 is a secreted protein that is deposited in the extracellular matrix (Konduri et al., 2001). Indeed, the transient knock-down of CD24 lead to an increased presence of TFPI-2 in the ECM of all three cell lines (Fig. 2C). Transient TFPI-2 depletion modulates the cell invasion of lung cancer cells (Iochmann et al., 2009). To study whether this was true also for the own cell lines, TFPI-2 was depleted by siRNA and invasion assays were carried out. A knock-down efficacy of 60-75% as measured by RT-PCR and Western blot was achieved (Fig. 2B, C) that, depending on the cell line, led to an increased invasion of 25-45% compared to control siRNA (Fig. 2D).

### Example 4: Knock-down of CD24 or TFPI-2 does not change MMP expression

Next, the question whether altered levels of MMPs contributed to the observed changes in cell invasion was addressed. The levels of MMP-2 and MMP-9 after transient depletion of CD24 by siRNA were determined, but no major changes were detected by qRT-PCR analysis (Fig. 3A). Similarly, depletion of TFPI-2 caused only minor changes except for a small increase of MMP-2 mRNA in A549 cells (Fig. 3B). Gelatin zymography analysis revealed detectable activity in HS683 cells but no changes occurred after CD24 or TFPI-2 knock-down (Fig. 3C). These results suggested that CD24 or TFPI-2 depletion does not lead to gross changes in MMP-2 and MMP-9 expression.

### Example 5: TFPI-2 overexpression inhibits cell invasion

These results so far suggested, that transient knock-down of CD24 augments TFPI-2 expression and reduces cell invasion. Thus, TFPI-2 modulation might be instrumental for the process of CD24-dependent invasion. This assumption agreed with the observation, that SNB19 glioblastoma cells did not show enhanced invasion after CD24 knock-down as these cells do not express detectable levels of TFPI-2 (Fig. 4A). Therefore, SNB19 cells were used to study the effect of TFPI-2 overexpression on cell invasion. As expected, the transient expression of TFPI-2 restored the ECM deposition in a dose dependent fashion (Fig. 4B) and led to a reduced cell invasion (Fig. 4C). These results extended earlier findings (Konduri et al., 2001) implying an important role of TFPI-2 in the regulation of tumor cell invasion.

### Example 6: CD24 overexpression suppressed TFPI-2 and augments cell invasion

Due to a genetic polymorphism in the GPI-anchor attachment site, CD24 can occur in two isoforms referred two as CD24-Ala or CD24-Val (Zhou et al., 2003). Previous work has suggested a role of the CD24 Ala/Val polymorphism for the progression of autoimmune disease such as multiple sclerosis (MS), and systemic lupus erythematosus (SLE) (Fang et al., 2010). Both forms were stably expressed in A 125 lung adenocarcinoma cells and expression was verified by FACS analysis (Fig. 5A). The expression of both forms reduced the amount of TFPI-2 in the ECM (Fig. 5B) and significantly augmented cell invasion into matrigel (Fig. 5C). These results suggested that both CD24 isoforms have the ability to influence TFPI-2 expression and cell invasion.

### Example 7: CD24 depletion affects c-Src activity

Since CD24 co-localizes with c-Src in membrane rafts, next, the level of Src phosphorylation after CD24 knock-down was determined in SKOV3ip and A549 cells. Whereas total Src protein levels were not different, Phospho-Src Y416 (active Src) was decreased and Phospho-Src Y527 (inactive Src) was increased following CD24 knock-down (Fig. 6A).
To establish a role of Src in TFPI-2 regulation, Src knock-down in SKOV3ip and A549 cells was used. Src depletion was between 50-60% in both cell lines (Fig. 10B). Up-regulation of TFPI-2 at the mRNA level was seen in SKOV3ip and to a weaker extent in A549 cells (Fig. 6B). The matrix deposition of TFPI-2 protein was clearly enhanced after Src knock-down in both cell lines (Fig. 6C). The transient depletion also led to decreased cell invasion for both cell lines (Fig. 6D). These results suggested that the CD24-Src axis played an important role in the regulation of TFPI-expression and cell invasion.

### Example 8: Analysis of CD24 and TFPI-2 in BrCa tissues

A TMA with 1174 primary breast cancers was analyzed for the expression of CD24 and TFPI-2. As expected, CD24 on its own was prognostic in this cohort: CD24high cases showed significantly shorter overall survival times than CD24low cases (Kaplan-Meyer analysis, Log-rank-test p=0.004). TFPI-2 also had a minor prognostic value with TFPI-2 low cases showing slightly shorter overall survival times, although significance was missed (Kaplan-Meyer analysis, Log-rank-test p=0.055). The data were then stratified in four groups: both low; CD24 low/TFPI-2 high; CD24 high/TFPI-2 low; or both high and representative staining examples are depicted in Fig. 7A. The analysis revealed that TFPI-2 expression was highest in CD24 negative samples and lowered with increasing CD24 expression (Fig. 7B). Patients with a CD24 low/TFPI-2 high phenotype showed significantly better survival compared to CD24 high/TFPI-2 low patients (Fig. 11C) (p=0.001). In addition, CD24 high cases with TFPI-2 low levels were significantly worse than CD24 high alone (p<0.001). However, this prognostic significance failed in a multivariate Cox model including patient age, pT category, nodal status and tumor grade (not shown).

### Example 9: CD24 knock-down affects cell adhesion and proliferation of cancer cell lines

The effects of transient knock-down of CD24 were investigated in a total of four tumor cell lines i.e. A549 lung carcinoma, SKOV3ip ovarian carcinoma, HS683 and SN19 glioblastoma cells. The efficacy of CD24 knock-down was investigated by Western blot analysis and FACS analysis of cell-surface CD24 (Fig. 8A and B) and was between 60-98%. Previous reports have shown that CD24 influences cell proliferation (Sagiv et al., 2008; Smith et al., 2006). It was found that the depletion of CD24 by siCD24 lead to a profound inhibition of cell proliferation in all cell lines (Fig. 8C). In agreement with previous reports (Smith et al., 2006) the loss of proliferation was accompanied by the induction of apoptosis as detected by annexin-V/PI staining (Fig. 8D). CD24 has been shown to play a role in cell adhesion and invasion (Baumann et al., 2005; Baumann et al., 2011; Hahne et al., 1994; Runz et al., 2008; Senner et al., 1999). Cell adhesion to immobilized ECM components such as laminin and the fibronectin subfragment FN-40, which supports only α4β1 mediated cell adhesion was examined. It was observed that CD24 knock-down inhibited cell adhesion of HS683 and SN 19 glioblastoma cells to FN-40 and laminin (Fig. 9A). Moreover, the CD24 knock-down diminished the phosphorylation of FAK at the Src-phosphorylation site (Fig. 9B). Additional experiments herein showed, that the siCD24 mediated knock-down reduced matrigel invasion in three of the four cell lines. These results demonstrate that CD24 expression contributes to essential cellular functions of tumor cells such as proliferation, adhesion and invasion.

### Example 10: CD24 affects gene regulation in human tumor cell lines

In colorectal and pancreatic carcinoma cells CD24 controls gene expression by an unknown mechanism (Sagiv et al., 2008). To verify this in SKOV3ip cells, DNA microarray analysis was used to identify genes affected by siCD24 knock-down. It was found that appr. 200 genes were more than 1.5 fold regulated.. The present inventors chose 4 genes (OPG, MMP7, STC-1 and STAT3) based on an estimated role in cell invasion for further analysis by qPCR and found them to be either up-regulated (OPG, MMP7, STC-1) or down-regulated (STAT3) by CD24 depletion (Fig. 10A). Strikingly, the present inventors observed that the selected genes were similarly regulated in the other cell lines i.e. HS683, A549 and SNB19 by CD24 knock-down (Fig. 10A) and identified the transcription factor STAT3 as a down-regulated gene product (Fig. 10A). To exclude off-target effects by siCD24, four additional siRNAs (siCD24-1-4) were tested. The depletion of CD24 was observed to a variable extent by all additional siRNAs and was associated with a reduction of STAT3 expression (Fig. 10B). These data suggest that the observed STAT3 regulation is CD24 specific.

### Example 11: CD24 regulates STAT3 transcriptional activity

STAT3 was initially described as a key component linking cytokine signaling to transcriptional regulation under physiological conditions, but is now known to play also an important role in cancer (Dauer et al., 2005; Yu et al., 2009). It was observed that the knockdown of CD24 significantly decreased the expression and phosphorylation of STAT3 in all the cell lines analyzed (Fig. 10C left lane). Thus, it was investigated whether CD24 depletion could alter STAT3 transcriptional activity. Indeed, the knock-down of CD24 led to significantly decreased activity in a STAT3-specific luciferase reporter assay (Fig. 10D) and was at a similar level as after STAT3 depletion (Fig. 10D). In cancer cells, STAT3 signaling is often constitutively active and many genes were described to depend on STAT3 activity (Dauer et al., 2005). To analyze whether CD24 and STAT3 depletion have similar effects, the regulation of cyclin D1, survivin and MCL-1 was studied, which all were found to be target genes of STAT3 in tumor cells (Dauer et al., 2005). Indeed, the knock-downs of CD24 and STAT3 caused similar effects on the expression of the selected genes (Fig. 11A).

### Example 12: CD24 affected genes are STAT3 target genes

To test whether the CD24-dependent genes identified in the initial screening (see Fig. 10A) might also depend on STAT3, the present inventors studied the regulation of the above gene signature (MMP-7, OPG, STC-1) after STAT3 depletion. Indeed, several of the genes such as STC-1 in SKOV3ip cells, or MMP7 in A549 cells were regulated by STAT3 knock-down (Fig. 11B). Thus, several of the genes identified by CD24 knock-down appeared to be STAT3 target genes in the respective cell line.

### Example 13: Regulation of STAT3-dependent genes requires Src activity

As described herein, Src-Y416 phosphorylation (active Src) was significantly diminished after siCD24 knock-down in A549 and SKOV3ip cells. Consistent with a loss of activity, Src-Y527 (inactive Src) phosphorylation was increased. Since Src activates STAT3 (Cao et al., 1996; Turkson et al., 1998) and FAK (Calalb et al., 1995; Schaller et al., 1994) it was examined whether Src is necessary for STAT3 activity in these cell lines. Src was depleted by specific siRNA and determined knock-down efficacy by q-PCR (Fig. 12A). Indeed, Src depletion altered the phosphorylation of STAT3 but STAT3 levels remained unaffected under these conditions (Fig. 12D). q-PCR analysis suggested that the Src knock-down also altered the expression of STAT3-dependent genes such as survivin, MMP-7, OPG and STC-1 in SKOV3ip and A549 cells (Fig. 12E). These results suggest that the CD24-Src link played an important role in the regulation of STAT3 transcriptional activity.

### Example 14: CD24 overexpression alters STAT3-dependent genes

To corroborate these findings, overexpression of CD24 in A125 lung adenocarcinoma cells was used andA125 cells stably transduced with CD24 showing either high (A125-CD24high) or low (A125-CD24low) expression (Mierke et al., 2011; Runz et al., 2008) were analyzed. The levels of CD24 expression in these cells are depicted in Fig. 13A. Western blot analysis revealed strong expression of phospho-STAT3-Y705, phospho-FAK-Y925 and phospho-Scr-Y416 (Fig. 13B). Importantly, the expression levels STAT3-dependent genes survivin and BCL-2 were both significantly up-regulated (Fig. 13C) whereas STC-1 was down-regulated in CD24-high expressing cells. Taken together, these studies support the CD24 know-down results and suggest an important role of CD24 in the regulation of STAT3.

### Example 15: mAb directed against CD24 affect tumor growth in vivo

A recent study has shown that CD24 drives lung metastasis formation of bladder cancer and might be a good candidate for a mAb-dependent therapy (Overdevest et al., 2011). It was analyzed whether the mAb SWA11 directed against CD24 is suitable to prevent tumor growth in xenotransplanted mice. In this therapy model, A549 human lung carcinoma cells were implanted s.c. into SCID/Beige mice and five injections of the SWA11 mAb were given over 37 days. The therapy with the mAb SWA11 led to a substantially reduced tumor load (Fig. 14A). The final tumor volume in SWA11 mAb treated animals was significantly decreased as compared to the isotype control-mAb treated animals (Fig. 14A). Similar observations were made in a second therapy model of CD24-positive BxPC3 pancreatic adenocarcinoma cells in NOD/SCID mice (Fig. 14B). Even more pronounced were the therapeutic effects of the mAb SWA11 application in the SKOV3ip ovarian carcinoma model where the tumor load was decreased by >80% as compared to the isotype control-mAb (Fig. 15). To reveal insights into the effector mechanisms, the status of Src phosphorylation was analyzed in tumor sections using immunofluorescence staining. To confirm that the therapeutic mAb had reached the tumor site, the sections were also stained with a Alexa488-conjugated pAb to mouse IgG. In both A549 and BxPC3 tumor tissue sections an increase in p-Src Y527 staining (inactive Src) in SWA11 mAb treated tumors compared to isotype control-mAb treated tumors was observed (Fig. 13C and D). This was confirmed by Western blot analysis of the tumor lysates (data not shown). To examine the influence of antibody therapy on gene expression, the present inventors analyzed tumor tissue by homogenization and extraction of mRNA for q-PCR analysis. By applying the STAT3-dependent gene signature established above, they observed significant gene regulation effects evoked by the mAb SWA11 treated tumor compared to the isotype control treated tumors (Fig. 14E and F). This was true for both A549 and BxPC3 mouse tumor models (Fig. 18E and F) and even for the SKOV3ip model (see Fig. 15). These results suggest that the siRNA-mediated CD24 depletion *in vitro* and the antibody treatment in vivo are i partly overlapping in their functional effects on STAT3 gene expression.

### REFERENCES

Ahmed MA, Jackson D, Seth R, et al. CD24 is upregulated in inflammatory bowel disease and stimulates cell motility and colony formation. Inflamm Bowel Dis 2009; 16 (5): 795-803.
Aigner S, Sthoeger ZM, Fogel M, et al. CD24, a mucin-type glycoprotein, is a ligand for P-selectin on human tumor cells. Blood 1997; 89 (9): 3385-95.
Aigner S, Ramos CL, Hafezi-Moghadam A, Lawrence MB, Friederichs J, Altevogt P et al. CD24 mediates rolling of breast carcinoma cells on P-selectin. Faseb J 1998; 12 (12): 1241-51.
Al-Hajj M, Wicha MS, Benito-Hernandez A, et al. Prospective identification of tumorigenic breast cancer cells. Proc Natl Acad Sci U S A 2003; 100 (7): 3983-8.
Baumann P, Cremers N, Kroese F, et al. CD24 expression causes the acquisition of multiple cellular properties associated with tumor growth and metastasis. Cancer Res 2005; 65 (23): 10783-93.
Baumann P, Thiele W, Cremers N, et al. CD24 interacts with and promotes the activity of c-src within lipid rafts in breast cancer cells, thereby increasing integrin-dependent adhesion. Cell Mol Life Sci 2011.
Brunton VG, Avizienyte E, Fincham VJ, Serrels B, Metcalf CA, 3rd, Sawyer TK et al (2005). Identification of Src-specific phosphorylation site on focal adhesion kinase: dissection of the role of Src SH2 and catalytic functions and their consequences for tumor cell behavior. Cancer Res 65: 1335-42.
Calalb MB, Polte TR, Hanks SK (1995). Tyrosine phosphorylation of focal adhesion kinase at sites in the catalytic domain regulates kinase activity: a role for Src family kinases. Mol Cell Biol 15: 954-63. Cao X, Tay A, Guy GR, Tan YH (1996). Activation and association of Stat3 with Src in v-Src-transformed cell lines. Mol Cell Biol 16: 1595-603.
Cao X, Geradts J, Dewhirst MW, et al. Upregulation of VEGF-A and CD24 gene expression by the tGLI transcription factor contributes to the aggressive behavior of breast cancer cells. Oncogene 2011.
Dauer DJ, Ferraro B, Song L, et al. Stat3 regulates genes common to both wound healing and cancer. Oncogene 2005; 24 (21): 3397-408.
Fang X, Zheng P, Tang J, et al. CD24: from A to Z. Cell Mol Immune) 2010; 7 (2): 100-3.
Friederichs J, Zeller Y, Hafezi-Moghadam A, et al. The CD24/P-selectin binding pathway initiates lung arrest of human A 125 adenocarcinoma cells. Cancer Res 2000; 60 (23): 6714-22.
Fukushima T, Tezuka T, Shimomura T, et al. Silencing of insulin-like growth factor-binding protein-2 in human glioblastoma cells reduces both invasiveness and expression of progression-associated gene CD24. J Biol Chem 2007; 282 (25): 18634-44.
Gast D, Riedle S, Issa Y, et al. The cytoplasmic part of L1-CAM controls growth and gene expression in human tumors that is reversed by therapeutic antibodies. Oncogene 2008; 27 (9): 1281-9.
Gaud G, Iochmann S, Guillon-Munos A, et al. TFPI-2 silencing increases tumour progression and promotes metalloproteinase I and 3 induction through tumour-stromal cell interactions. J Cell Mol Med 2009.
Guarino M. Src signaling in cancer invasion. J Cell Physiol 2010; 223 (1): 14-26.
Guo H, Lin Y, Zhang H, et al. Tissue factor pathway inhibitor-2 was repressed by CpG hypermethylation through inhibition of KLF6 binding in highly invasive breast cancer cells. BMC Mol Biol 2007; 8: 110.
Hahne M, Wenger RH, Vestweber D, et al. The heat-stable antigen can alter very late antigen 4-mediated adhesion. J Exp Med 1994; 179 (4): 1391-5.
Herman MP, Sukhova GK, Kisiel W, et al. Tissue factor pathway inhibitor-2 is a novel inhibitor of matrix metalloproteinases with implications for atherosclerosis. J Clin Invest 2001; 107 (9): 1117-26.
Hitosugi T, Sato M, Sasaki K, et al. Lipid raft specific knockdown of SRC family kinase activity inhibits cell adhesion and cell cycle progression of breast cancer cells. Cancer Res 2007; 67 (17): 8139-48.
Honeth G, Bendahl PO, Ringner M, Saal LH, Gruvberger-Saal SK, Lovgren K et al (2008). The CD44+/CD24- phenotype is enriched in basal-like breast tumors. Breast Cancer Res 10: R53.
Ilangumaran S, Ami S, van Echten-Deckert G, et al. Microdomain-dependent regulation of Lck and Fyn protein-tyrosine kinases in T lymphocyte plasma membranes. Mol Biol Cell 1999; 10 (4): 891-905.
Iochmann S, Blechet C, Chabot V, et al. Transient RNA silencing of tissue factor pathway inhibitor-2 modulates lung cancer cell invasion. Clin Exp Metastasis 2009; 26 (5): 457-67.
Jackson D, Waibel R, Weber E, et al. CD24, a signal-transducing molecule expressed on human B cells, is a major surface antigen on small cell lung carcinomas. Cancer Res 1992; 52 (19): 5264-70.
Kast C, Wang M, Whiteway M. The ERK/MAPK pathway regulates the activity of the human tissue factor pathway inhibitor-2 promoter. J Biol Chem 2003; 278 (9): 6787-94.
Kay R, Takei F, Humphries RK. Expression cloning of a cDNA encoding M1/69-J11d heat-stable antigens. J Immunol 1990; 145 (6): 1952-9.
Kay R, Rosten PM, Humphries RK. CD24, a signal transducer modulating B cell activation responses, is a very short peptide with a glycosyl phosphatidylinositol membrane anchor. J Immunol 1991; 147 (4): 1412-6.
Kedersha N, Anderson P. Stress granules: sites of mRNA triage that regulate mRNA stability and translatability. Biochem Soc Trans 2002; 30 (Pt 6): 963-9.
Kiefel H, Bondong S, Erbe-Hoffmann N, Hazin J, Riedle S, Wolf J et al (2010). L1CAM-integrin interaction induces constitutive NF-kappaB activation in pancreatic adenocarcinoma cells by enhancing IL-1beta expression. Oncogene 29: 4766-78.
Konduri SD, Tasiou A, Chandrasekar N, et al. Role of tissue factor pathway inhibitor-2 (TFPI-2) in amelanotic melanoma (C-32) invasion. Clin Exp Metastasis 2000; 18 (4): 303-8.
Konduri SD, Tasiou A, Chandrasekar N, et al. Overexpression of tissue factor pathway inhibitor-2 (TFPI-2), decreases the invasiveness of prostate cancer cells in vitro. Int J Oncol 2001; 18 (1): 127-31.
Konduri SD, Rao CN, Chandrasekar N, et al. A novel function of tissue factor pathway inhibitor-2 (TFPI-2) in human glioma invasion. Oncogene 2001; 20 (47): 6938-45.
Koppikar P, Choi SH, Egloff AM, et al. Combined inhibition of c-Src and epidermal growth factor receptor abrogates growth and invasion of head and neck squamous cell carcinoma. Clin Cancer Res 2008; 14 (13): 4284-91.
Kristiansen G, Sammar M, Altevogt P. Tumour biological aspects of CD24, a mucin-like adhesion molecule. J Mol Histol 2004; 35 (3): 255-62.
Kristiansen G, Machado E, Bretz N, et al. Molecular and clinical dissection of CD24 antibody specificity by a comprehensive comparative analysis. Lab Invest 2010; 90 (7): 1102-16.
Kristiansen G, Rose M, Geisler C, et al. Endogenous myoglobin in human breast cancer is a hallmark of luminal cancer phenotype. Br J Cancer 2010; 102 (12): 1736-45.
Lee TK, Castilho A, Cheung VC, Tang KH, Ma S, Ng IO (2011). CD24(+) liver tumor-initiating cells drive self-renewal and tumor initiation through STAT3-mediated NANOG regulation. Cell Stem Cell 9: 50-63.
Lim SC (2005). CD24 and human carcinoma: tumor biological aspects. Biomed Pharmacother 59 Suppl 2: 5351-4.
Lo HW, Zhu H, Cao X, et al. A novel splice variant of GLI1 that promotes glioblastoma cell migration and invasion. Cancer Res 2009; 69 (17): 6790-8.
Marotta LL, Almendro V, Marusyk A, Shipitsin M, Schemme J, Walker SR et al (2011). The JAK2/STAT3 signaling pathway is required for growth of CD44+CD24- stem cell-like breast cancer cells in human tumors. J Clin Invest 121.
Mierke CT, Bretz N, Altevogt P. Contractile forces contribute to increased GPI-anchored receptor CD24 facilitated cancer cell invasion. J Biol Chem 2011; doi:10.1074/jbc.M111.245183 in press.
Nielsen PJ, Lorenz B, Muller AM, Wenger RH, Brombacher F, Simon M et al (1997). Altered erythrocytes and a leaky block in B-cell development in CD24/HSA-deficient mice. Blood 89: 1058-67.
Overdevest JB, Thomas S, Kristiansen G, et al. CD24 offers a therapeutic target for control of bladder cancer metastasis based on a requirement for lung colonization. Cancer Res 2011; 71 (11): 3802-11.
Pfeifer M, Schirmer U, Geismann C, Schafer H, Sebens S, Altevogt P (2010). L1CAM expression in endometrial carcinomas is regulated by usage of two different promoter regions. BMC Mol Biol 11: 64.
Riedle S, Kiefel H, Gast D, et al. Nuclear translocation and signalling of L1-CAM in human carcinoma cells requires ADAM10 and presenilin/gamma-secretase activity. Biochem J 2009; 420 (3): 391-402.
Runz S, Keller S, Rupp C, et al. Malignant ascites-derived exosomes of ovarian carcinoma patients contain CD24 and EpCAM. Gynecol Oncol 2007; 107 (3): 563-71.
Runz S, Mierke CT, Joumaa S, et al. CD24 induces localization of beta1 integrin to lipid raft domains. Biochem Biophys Res Commun 2008; 365 (1): 35-41.
Sagiv E, Starr A, Rozovski U, et al. Targeting CD24 for treatment of colorectal and pancreatic cancer by monoclonal antibodies or small interfering RNA. Cancer Res 2008; 68 (8): 2803-12.
Sammar M, Aigner S, Hubbe M, et al. Heat-stable antigen (CD24) as ligand for mouse P-selectin. Int Immunol 1994; 6 (7): 1027-36.
Sammar M, Gulbins E, Hilbert K, et al. Mouse CD24 as a signaling molecule for integrin-mediated cell binding: functional and physical association with src-kinases. Biochem Biophys Res Commun 1997; 234 (2): 330-4.
Schabath H, Runz S, Joumaa S, et al. CD24 affects CXCR4 function in pre-B lymphocytes and breast carcinoma cells. J Cell Sci 2006; 119 (Pt 2): 314-25.
Schaller MD, Hildebrand JD, Shannon JD, Fox JW, Vines RR, Parsons JT (1994). Autophosphorylation of the focal adhesion kinase, pp125FAK, directs SH2-dependent binding of pp60src. Mol Cell Biol 14: 1680-8.
Senner V, Sturm A, Baur I, et al. CD24 promotes invasion of glioma cells in vivo. J Neuropathol Exp Neurol 1999; 58 (8): 795-802.
Sierko E, Wojtukiewicz MZ, Kisiel W. The role of tissue factor pathway inhibitor-2 in cancer biology. Semin Thromb Hemost 2007; 33 (7): 653-9.
Simons K, Gerl MJ. Revitalizing membrane rafts: new tools and insights. Nat Rev Mol Cell Biol 2010; 11 (10): 688-99.
Smith SC, Oxford G, Wu Z, et al. The metastasis-associated gene CD24 is regulated by Ral GTPase and is a mediator of cell proliferation and survival in human cancer. Cancer Res 2006; 66 (4): 1917-22.
Stefanova I, Horejsi V, Ansotegui IJ, et al. GPI-anchored cell-surface molecules complexed to protein tyrosine kinases. Science 1991; 254 (5034): 1016-9.
Stoeck A, Gast D, Sanderson MP, Issa Y, Gutwein P, Altevogt P (2007). L1-CAM in a membrane-bound or soluble form augments protection from apoptosis in ovarian carcinoma cells. Gynecol Oncol 104: 461-9.
Taniuchi K, Nishimori 1, Hollingsworth MA. Intracellular CD24 Inhibits Cell Invasion by Posttranscriptional Regulation of BART through Interaction with G3BP. Cancer Res 2011; 71 (3): 895-905.
Theurillat JP, Ingold F, Frei C, et al. NY-ESO-1 protein expression in primary breast carcinoma and metastases: correlation with CD8+ T-cell and CD79a+ plasmacytic/B-cell infiltration. Int J Cancer 2007; 120 (11): 2411-7.
Turkson J, Bowman T, Garcia R, Caldenhoven E, De Groot RP, Jove R (1998). Stat3 activation by Src induces specific gene regulation and is required for cell transformation. Mol Cell Biol 18: 2545-52.
Varma R, Mayor S. GPI-anchored proteins are organized in submicron domains at the cell surface. Nature 1998; 394 (6695): 798-801.
Wang W, Wang X, Peng L, et al. CD24-dependent MAPK pathway activation is required for colorectal cancer cell proliferation. Cancer Sci 2010; 101 (1): 112-9.
Weber E, et al., Antibodies to the protein core of the small cell lung cancer workshop antigen cluster-w4 and to the leucocyte workshop antigen CD24 recognize the same short protein sequence leucine-alanine-proline. Clin Exp Immunol. 1993 Aug;93(2):279-85
Wenger RH, Ayane M, Bose R, et al. The genes for a mouse hematopoietic differentiation marker called the heat-stable antigen. Eur J Immunol 1991; 21 (4): 1039-46.
Wolterink S, Moldenhauer G, Fogel M, et al. Therapeutic antibodies to human L1CAM: functional characterization and application in a mouse model for ovarian carcinoma. Cancer Res 2010; 70 (6): 2504-15.
Woodward WA, Sulman EP. Cancer stem cells: markers or biomarkers? Cancer Metastasis Rev 2008; 27 (3): 459-70.
Yeatman TJ. A renaissance for SRC. Nat Rev Cancer 2004; 4 (6): 470-80.
Yu H, Pardoll D, Jove R (2009). STATs in cancer inflammation and immunity: a leading role for STAT3. Nat Rev Cancer 9: 798-809.
Zam JA, Zimmermann SM, Pass MK, et al. Association of CD24 with the kinase c-fgr in a small cell lung cancer cell line and with the kinase lyn in an erythroleukemia cell line. Biochem Biophys Res Commun 1996; 225 (2): 384-91.
Zhou Q, Rammohan K, Lin S, et al. CD24 is a genetic modifier for risk and progression of multiple sclerosis. Proc Natl Acad Sci U S A 2003; 100 (25): 15041-6.
Zhou C, Cunningham L, Marcus Al, et al. Arl2 and Arl3 regulate different microtubule-dependent processes. Mol Biol Cell 2006; 17 (5): 2476-87.

## Claims

1. A method for determining whether a subject is susceptible to the treatment of a neoplastic condition with a CD24 inhibitor comprising the step(s) of
i) treating a sample of said subject, particularly of a neoplasm of said subject with a CD24 inhibitor;
ii) determining
a) the level of expression of STAT3,
b) the level of phosphorylation of STAT3,
c) the level of phosphorylation of FAK,
d) the level of phosphorylation of src at Y416,
e) the level of phosphorylation of src at Y527,
f) the level of expression of OPG,
g) the level of expression of STC-1,
h) the level of expression of MMP-7,
and/or
j) the level of expression of TFPI-2;
wherein
a) a decreased level of expression of STAT3,
b) a decreased level of phosphorylation of STAT3,
c) a decreased level of phosphorylation of FAK,
d) a decreased level of phosphorylation of src at Y416,
e) an increased level of phosphorylation of src at Y527,
f) an increased level of expression of OPG,
g) an increased level of expression of STC-1,
h) an increased level of expression of MMP-7,
and/or
j) an increased level of expression of TFPI-2
in said sample is indicative of the subject being susceptible to said treatment.

2. The method of claim 1, wherein said increased and/or decreased level(s) is/are verified by means of a control in the absence of a treatment with a CD24 inhibitor.

3. The method of any of claims 1 or 2, further comprising the treatment of said sample with an anti-tumor agent, wherein it is determined whether said subject is susceptible to a treatment with a combination of said CD24 inhibitor and said antitumor agent.

4. A method for determining whether a subject suffering from a neoplastic condition has increased chances of a beneficial outcome, the method comprising at least two of the below step(s) of
a) determining the level of expression of STAT3,
b) determining the level of phosphorylation of STAT3,
c) determining the level of phosphorylation of FAK,
d) determining the level of phosphorylation of src at Y416,
e) determining the level of phosphorylation of src at Y527,
f) determining the level of expression of OPG,
g) determining the level of expression of STC-1,
h) determining the level of expression of MMP-7,
j) determining the level of expression of TFPI-2;
and/or
k) determining the level of expression of CD24;
in a sample of said subject,
wherein
a) a decreased level of expression of STAT3,
b) a decreased level of phosphorylation of STAT3,
c) a decreased level of phosphorylation of FAK,
d) a decreased level of phosphorylation of src at Y416,
e) an increased level of phosphorylation of src at Y527,
f) an increased level of expression of OPG,
g) an increased level of expression of STC-1,
h) an increased level of expression of MMP-7,
j) an increased level of expression of TFPI-2
and/or
k) a decreased level of expression of CD24;
is indicative of the subject having increased chances of a beneficial outcome.

5. The method of claim 4, said increased and/or decreased level(s) is/are verified by means of a controls, which are selected from the following controls
for said levels defined in items a), f), g), h), j) and k) determining the level of expression of one or more house-keeping genes, particularly wherein said house-keeping gene is beta-actin;
for said level defined in item b) determining the level of expression of STAT3; for said level defined in item c) determining the level of expression of FAK; and for said levels defined in items d) and e) determining the level of expression of src,

6. A CD24 inhibitor for use in a method for treating a neoplastic condition in a subject susceptible to said treatment, wherein said subject has been determined to be susceptible to said treatment by means of a method according to claim 1 or 2.

7. A CD24 inhibitor for use in a method for treating a neoplastic condition in a subject susceptible to said treatment, wherein said method further comprises the administration of an anti-tumor agent, and wherein said subject has been determined to be susceptible to said treatment by means of a method according to claim 3.

8. A CD24 inhibitor for use in a method for treating a neoplastic condition in a subject, said method comprising
i) treating a sample of said subject, particularly of a neoplasm of said subject, with a CD24 inhibitor;
ii) determining
a) the level of expression of STAT3,
b) the level of phosphorylation of STAT3,
c) the level of phosphorylation of FAK,
d) the level of phosphorylation of src at Y416,
e) the level of phosphorylation of src at Y527,
f) the level of expression of OPG,
g) the level of expression of STC-1,
h) the level of expression of MMP-7,
and/or
j) the level of expression of TFPI-2;
and
ii) administering a CD24 inhibitor to said subject provided that said treated sample shows
a) a decreased level of expression of STAT3,
b) a decreased level of phosphorylation of STAT3,
c) a decreased level of phosphorylation of FAK,
d) a decreased level of phosphorylation of src at Y416,
e) an increased level of phosphorylation of src at Y527,
f) an increased level of expression of OPG,
g) an increased level of expression of STC-1,
h) an increased level of expression of MMP-7,
and/or
j) an increased level of expression of TFPI-2
in said sample.

9. The CD24 inhibitor for use according to claim 8, wherein said increased and/or decreased level(s) is/are verified by means of a control in the absence of a CD24 inhibitor.

10. The CD24 inhibitor for use according to any of claims 8 or 9, wherein said method further comprises the treatment of said sample with an anti-tumor agent, wherein it is determined whether said subject is susceptible to a treatment with a combination of said CD24 inhibitor and said antitumor agent, and wherein said method further comprises the administration of said ant-tumor agent to said subject.

11. The method of any one of claims 1 to 3 or the CD24 inhibitor for use of any one of claims 6 to 10, wherein said CD24 inhibitor
i) is an anti-CD24 antibody, particularly a monoclonal anti-CD24 antibody, more particularly a monoclonal anti-CD24 inhibitor recognizing the LAP sequence in CD24;
ii) is a CD24 siRNA.

12. The method or CD24 inhibitor for use of any one of claims 1 to 11, wherein said neoplastic condition is selected from the group consisting of astrocytoma, B-cell lymphoma, brain carcinoma, breast adenocarcinoma, breast carcinoma, Burkitt's B-cell lymphoma, cervical adenocarcinoma, colon adenocarcinoma, colorectal adenocarcinoma, colorectal carcinoma, glioblastoma, hepatocellular carcinoma, hepatoma and pancreatic carcinoma, leukaemia, large cell lung cancer, lung adenocarcinoma, lung carcinoma, lung mesothelioma, lung squamous cell carcinoma, melanoma, neuroblastoma, non small cell and small cell lung cancer (NSCLC and SCLC), oesophageal cancer, ovarian adenocarcinoma, pancreatic adenocarcinoma, pancreatic carcinoma, prostate adenocarcinoma, prostate carcinoma, rectal adenocarcinoma, renal cell adenocarcinoma, small cell lung cancer (SCLC), T-cell lymphoma, thyroid cancer, and uterine cancer;
particularly wherein said neoplastic condition is selected from the group consisting of brain carcinoma, breast adenocarcinoma, breast carcinoma, large cell lung cancer, lung adenocarcinoma, lung carcinoma, lung mesothelioma, lung squamous cell carcinoma, non small cell and small cell lung cancer, and ovarian adenocarcinoma.

13. The method of any of claims 3, 11 or 12 or the composition for use of any one of claims 6 to 12, wherein said anti-tumor agent is selected from the group consisting of a chemotherapeutic agent and a radiotherapeutic agent.
